# EUROPEAN PATENT SPECIFICATION

(11) **EP 1 827 427 B1**
(45) Date of publication and mention of the grant of the patent: **23.07.2008**
(21) Application number: 05850037.2
(22) Date of filing: 06.12.2005
(51) Int. Cl.: A61K 31/423, C07D 263/58, C07D 413/12, A61P 25/00

(54) **ARYLOXYETHYLAMINE DERIVATIVES WITH A COMBINATION OF PARTIAL DOPAMINE-D2 RECEPTOR AGONISM AND SEROTONIN REUPTAKE INHIBITION**
ARYLOXYETHYLAMINDERIVATE MIT EINER KOMBINATION AUS PARTIELLEM DOPAMIN-D2-REZEPTORAGONISMUS UND SEROTONIN-WIEDERAUFNAHMEHEMMUNG
DERIVÉS D'ARYLOXETHYLAMINE ET DE PHÉNYLPIPÉRAZINE COMME AGONISTES PARTIELS DU RÉCEPTEUR D2 DE LA DOPAMINE ET INHIBITEURS DE RECAPTAGE DE LA SÉROTONINE

(30) Priority: 08.12.2004 US 634074 P; 08.12.2004 EP 04106394
(43) Date of publication of application: 05.09.2007
(73) Proprietor: Solvay Pharmaceuticals B.V., 1381 CP Weesp (NL)
(72) Inventor: FEENSTRA, Roelof, W., NL-1381 CP Weesp (NL); STOIT, Axel, NL-1381 CP Weesp (NL); TERPSTRA, Jan-Willem, NL-1381 CPWeesp (NL); PRAS-RAVES, Maria, L., NL-1381 CP Weesp (NL); MCCREARY, Andrew, C., NL-1381 CP Weesp (NL); VAN VLIET, Bernard, J., NL-1381CP Weesp (NL); HESSELINK, Mayke, B., NL-1381 CP Weesp (NL); KRUSE, Cornelis, G., NL-1381 CP Weesp (NL); VAN SCHARRENBURG, Gustaaf, J.M., NL-1381 CP Weesp (NL)
(74) Representative: Verhage, Marinus
(86) International application number: PCT/EP2005/056505
(87) International publication number: WO 2006/061376

(56) References cited:
- WO-A-00/43382
- WO-A-01/14330
- WO-A-99/51575
- WO-A-20/04052886
- US-A- 5 464 834

## Description

The present invention relates to a group of novel aryloxyethylamine derivatives with a dual mode of action: serotonin reuptake inhibition and partial agonism on dopamine-D₂ receptors. The invention also relates to the use of a compound disclosed herein for the manufacture of a medicament giving a beneficial effect. A beneficial effect is disclosed herein or apparent to a person skilled in the art from the specification and general knowledge in the art. The invention also relates to the use of a compound of the invention for the manufacture of a medicament for treating or preventing a disease or condition. More particularly, the invention relates to a new use for the treatment of a disease or condition disclosed herein or apparent to a person skilled in the art from the specification and general knowledge in the art. In embodiments of the invention specific compounds disclosed herein are used for the manufacture of a medicament useful in the treatment of disorders in which dopamine-D₂ receptors and serotonin reuptake sites are involved, or that can be treated via manipulation of those targets.

Compounds with a dual action as dopamine-D₂ antagonists and serotonin reuptake inhibitors are known from WO 00/023441, WO 00/069424 and WO 01/014330. This combination of activities is useful for the treatment of schizophrenia and other psychotic disorders: it enables a more complete treatment of all disease symptoms (e.g. positive symptoms and negative symptoms).

The goal of the present invention was to provide further compounds with a dual action as partial dopamine-D₂ agonists and serotonin reuptake inhibitors.

The invention relates to a group of novel compounds of the formula (1):

**Z-T-Ar** (1)

wherein:
Z is a fragment of the general formula (2) 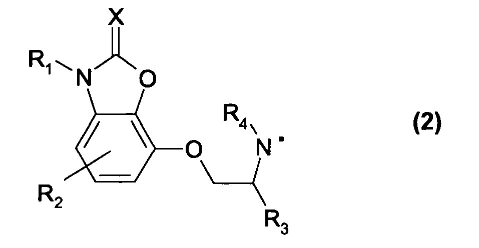 wherein: X = S or O,
   R₁ is H, (C₁-C₆)alkyl, CF₃, CH₂CF₃, OH or O-(C₁-C₆)alkyl
   R₂ is H, (C₁-C₆)alkyl, halogen or cyano
   R₃ is H or (C₁-C₆)alkyl
   R₄ is H, (C₁-C₆)alkyl optionally substituted with a halogen atom
T is a saturated or unsaturated carbon chain of 2 -7 atoms, wherein one carbon atom may be replaced with a nitrogen atom, optionally substituted with an (C₁-C₃)- alkyl, CF₃ or CH₂CF₃ group, or an oxygen atom or a sulphur atom, and which chain is optionally substituted with one or more substituents selected from the group consisting of (C₁-C₃)alkyl, (C₁-C₃)alkoxy, halogen, cyano, trifluoromethyl, OCF₃, SCF₃, OCHF₂ and nitro,
Ar is selected from the groups: 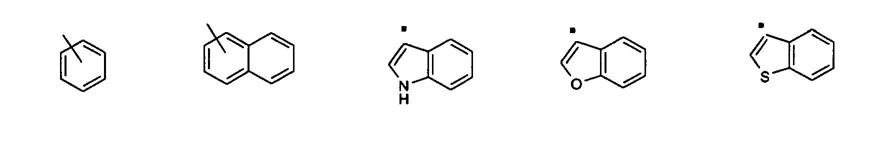 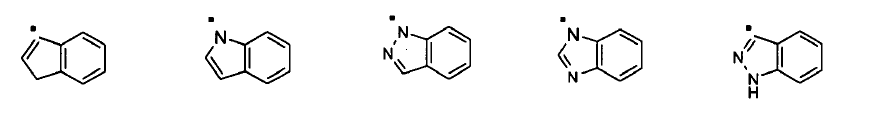
which Ar group is optionally further substituted with one or more substituents selected from the group consisting of (C₁-C₃)alkyl, (C₁-C₃)alkoxy, halogen, cyano, trifluoromethyl, OCF₃, SCF₃ OCHF₂ and nitro,
and in which Ar groups that contain a five-membered ring, the double bond in the five-membered ring may be saturated,
and tautomers, stereoisomers and N-oxides thereof, as well as pharmacologically acceptable salts, hydrates and solvates of said compounds of formula (1) and its tautomers, stereoisomers and N-oxides.

In formula (2) the dot on the N-atom is the attachment point of the group 'T-Ar'. In the groups 'Ar', the dot represents the attachement point of group 'T'.

In the description of the substituents the abbreviation 'alkyl(C₁₋₃)' means 'methyl, ethyl, n-propyl or isopropyl'.

Prodrugs of the compounds mentioned above are in the scope of the present invention. Prodrugs are therapeutic agents which are inactive per se but are transformed into one or more active metabolites. Prodrugs are bioreversible derivatives of drug molecules used to overcome some barriers to the utility of the parent drug molecule. These barriers include, but are not limited to, solubility, permeability, stability, presystemic metabolism and targeting limitations (Medicinal Chemistry: Principles and Practice, 1994, Ed.: F. D. King, p. 215; J. Stella, "Prodrugs as therapeutics", Expert Opin. Ther. Patents, 14(3), 277-280, 2004; P. Ettmayer et al., "Lessons learned from marketed and investigational prodrugs", J.Med.Chem., 47, 2393-2404, 2004). Pro-drugs, i.e. compounds which when administered to humans by any known route, are metabolised to compounds having formula (1), belong to the invention. In particular this relates to compounds with primary or secondary amino or hydroxy groups. Such compounds can be reacted with organic acids to yield compounds having formula (1) wherein an additional group is present which is easily removed after administration, for instance, but not limited to amidine, enamine, a Mannich base, a hydroxyl -methylene derivative, an O-(acyloxy-methylene carbamate) derivative, carbamate, ester, amide or enaminone.

N-oxides of the compounds mentioned above are in the scope of the present invention. Tertiary amines may or may not give rise to N-oxide metabolites. The extend to what N-oxidation takes place varies from trace amounts to a near quantitative conversion. N-oxides may be more active than their corresponding tertiary amines or less active. Whilst N-oxides are easily reduced to their corresponding tertiary amines by chemical means, in the human body this happens to varying degrees. Some N-oxides undergo nearly quantitative reductive conversion to the corresponding tertiary amines, in other cases the conversion is a mere trace reaction or even completely absent. (M.H. Bickel: "The pharmacology and Biochemistry of N-oxides", Pharmaco-logical Reviews, 21 (4), 325 - 355, 1969).

It has been found that the compounds according to the invention show high affinity for both the dopamine D₂ receptor and the serotonin reuptake site. The compounds show activity at dopamine D₂ receptors with varying degree of agonism. All of the compounds show activity as inhibitors of serotonin reu ptake, as they potentiate 5-HTP induced behaviour in mice (B.L. Jacobs., 'An animal behaviour model for studying central serotonergic synapses', Life Sci., 1976, 19(6), 777-785).

In contrast to the use of full dopamine-D₂ receptor agonists or antagonists, the use of partial dopamine-D₂ receptor agonists offers a dynamic medication that self-adjusts on a moment-to-moment basis to the endogenous state of the patient. Thus, it provides the desired flexible modulation of the dopamine system and avoidance of the many adverse effects caused either by treatment using full dopamine-D₂ receptor agonists like bromocriptine (hallucinations, nausea, vomiting, dyskinesia, orthostatic hypotension, somnolescence) or full dopamine -D₂ receptor antagonists like haloperidol (emotional blunting, dysphoria, tardive dyskinesia). Because of these many adverse effects, full agonists and antagonists have found only very limited use in the therapy of depressive and anxiety disorders. Partial dopamine-D₂ receptor agonists not only show a flexible modulation and a favourable side-effect profile, they also have a pronounced anxiolytic profile in relevant animal models (Drugs of the Future 2001, 26(2): 128-132).

Partial dopamine-D₂ receptor agonists, according to the present invention, are compounds that - when tested in a concentration response range - achieve activation in the functional cAMP cell based assay (as described below). Partial dopamine-D₂ receptor agonists will act as an agonist in cases when the endogenous synaptic tone of dopamine is low, or in the the presence of a full dopamine-D₂ receptor antagonist, and will act as an antagonist in cases when the endogenous synaptic tone of dopamine is high, or in the presence of a full dopamine D₂ receptor agonist. Like full agonists, partial dopamine-D₂ receptor agonists in general are active in sensitized systems. They induce contralateral turning in rats with unilateral 6-hydroxy-dopamine (6-OHDA) lesions in the substantia nigra pars compacta. In MPTP-treated common marmosets they produce potent and long-lasting reversal of motor symptoms (Drugs of the Future 2001, 26(2): 128-132). In contrast to full agonists, however, partial dopamine-D₂ agonists are substantially less active in non-sensitized systems: they hardly reverse reserpine induced hypolocomotion in rats.

For the treatment of CNS disorders involving an overactive dopaminergic system a pharmaceutical preparation combining partial dopamine-D₂ receptor agonistic activity having low intrinsic functional activity with serotonin reuptake inhibitory activity is recommended. In case of a disorder involving dopamine insufficiency a pharmaceutical preparation combining partial dopamine -D₂ receptor agonistic activity with high intrinsic functional activity and serotonin reuptake activity according to the invention has considerable advantages.

Disorders characterized by dynamic fluctuations in dopamine neurotransmission like bipolar depression and addiction will profit in particular from the flexible adjustment of the dopamine system by the partial dopamine-D₂ receptor agonists in the pharmaceutical preparation. Combining this "dopaminergic neurotransmission stabilizing" activity with serotonin reuptake inhibitory activity will enhance antidepressive and anxiolytic efficacy. The compounds can be used for the treatment of affections or diseases of the central nervous system caused by disturbances in the dopaminergic and serotonergic systems, for example: aggression, anxiety disorders, autism, vertigo, depression, disturbances of cognition or memory, Parkinson's disease, and in particular schizophrenia and other psychotic disorders.

Pharmaceutically acceptable salts may be obtained using standard procedures well known in the art, for example by mixing a compound of the present invention with a suitable acid, for instance an inorganic acid such as hydrochloric acid, or with an organic acid.

### PHARMACEUTICAL PREPARATIONS

The compounds of the invention can be brought into forms suitable for administration by means of usual processes using auxillary substances such as liquid or solid carrier material. The pharmaceutical compositions of the invention may be administered enterally, orally, parenterally (intramuscularly or intravenously), rectally or locally (topically). They can be administered in the form of solutions, powders, tablets, capsules (including microcapsules), ointments (creams or gel) or suppositories. Suitable excipients for such formulations are the pharmaceutically customary liquid or solid fillers and extenders, solvents, emulsifiers, lubricants, flavorings, colorings and/or buffer substances. Frequently used auxillary substances which may be mentioned are magnesium carbonate, titanium dioxide, lactose, mannitol and other sugars, talc, lactoprotein, gelatin, starch, cellulose and its derivatives, animal and vegetable oils such as fish liver oil, sunflower, groundnut or sesame oil, polyethylene glycol and solvents such as, for example, sterile water and mono- or polyhydric alcohols such as glycerol.

Compounds of the present invention are generally administered as pharmaceutical compositions which are important and novel embodiments of the invention because of the presence of the compounds, more particularly specific compounds disclosed herein. Types of pharmaceutical compositions that may be used include but are not limited to tablets, chewable tablets, capsules, solutions, parenteral solutions, suppositories, suspensions, and other types disclosed herein or apparent to a person skilled in the art from the specification and general knowledge in the art. In embodiments of the invention, a pharmaceutical pack or kit is provided comprising one or more containers filled with one or more of the ingredients of a pharmaceutical composition of the invention. Associated with such container(s) can be various written materials such as instructions for use, or a notice in the form prescribed by a governmental agency regulating the manufacture, use or sale of pharmaceuticals products, which notice reflects approval by the agency of manufacture, use, or sale for human or veterinary administration.

### PHARMACOLOGICAL METHODS

### In vitro affinity for dopamine-D₂ receptors

Affinity of the compounds for dopamine-D₂ receptor s was determined using the receptor binding assay described by I. Creese, R. Schneider and S.H. Snyder: "[3H]-Spiroperidol labels dopamine receptors in rat pituitary and brain", Eur.J.Pharmacol., 46, 377 - 381, 1977.

### In vitro affinity for serotonin reuptake sites

Affinity of the compounds for serotonin reuptake sites was determined using the receptor binding assay described by E. Habert et al.,: "Characterisation of [3H]-paroxetine binding to rat cortical membranes", Eur.J.Pharmacol., 118, 107-114, 1985.

### Inhibition of forskolin-induced [³H]-cAMP accumulation

The *in vitro* functional activity at dopamine-D₂ receptors, including the intrinsic activity (ε) of the compounds of the invention was measured by their ability to inhibit forskolin-induced [³H]-cAMP accumulation.

Human dopamine D_{2,L} receptors were cloned in fibroblast cell line CHO-K1 cells and obtained from Dr. Grandy, Vollum Institute, Portland, Oregon, USA. CHO cells were grown in a Dulbecco's modified Eagle's medium (DMEM) culture medium, supplemented with 10% heat-inactivated fetal calf serum, 2 mM glutamine, 1 mM pyruvate, 5000 units/ml penicillin, 5000 µg/ml streptomycin and 200 µg/ml G-418 at 37 °C in 93% air/7% CO₂. For incubation with test compounds, confluent cultures grown in 24 wells plates were used. Each condition or substance was routinely tested in quadruplicate. Cells were loaded with 1 µCi [³H]-adenine in 0.5 ml medium/well. After 2 hours, cultures were washed with 0.5 ml PBS containing 1 mM of the phosphodiesterase inhibitor isobutylmethylxanthine (IBMX) and incubated for 20 min with 0.5 ml PBS containing 1 mM IBMX and forskolin with or without test compound. After aspiration the reaction was stopped with 1 ml trichloroacetic acid 5% (w/v). The [³H]-ATP and [³H]-cAMP formed in the cellular extract were assayed as described by Solomon Y, Landos C, Rodbell M, 1974, A highly selective adenylyl cyclase assay, Anal Biochem 58:541-548 and Weiss S, Sebben M, Bockaert JJ, 1985, Corticotropin-peptide regulation of intracellular cyclic AMP production in cortical neurons in primary culture, J Neurochem 45:869-874. 0.8 ml Extract was passed over Dowex (50WX-4 200-400 mesh) and aluminumoxide columns, eluted with water and 0.1 M imidazole (pH=7.5). Eluates were mixed with 7 ml Insta -gel and radioactivity was counted with a liquid scintillation counter. The conversion of [ ³H]-ATP into [³H]-cAMP was expressed as the ratio in percentage radioactivity in the cAMP fraction as compared to combined radioactivity in both cAMP and ATP fractions, and basal activity was subtracted to correct for spontaneous activity.

Test compounds were obtained as 10 mM stock solutions in 100% DMSO, and diluted in PBS/IBMX to final concentrations. Typically, compounds were used in concentrations that ranged from 10⁻¹⁰M to 10⁻⁵M. From quadruplicate data counts, the mean was taken as an estimate for drug -induced, receptor-mediated effects at specified second messenger accumulation, expressed as percentage of control values (forskolin-stimulated cAMP accumulation, subtracted by basal activity). By using the non-linear curve-fitting program INPLOT or the Excel-add-in XL-Fit, mean values were plotted against drug concentration (in molar) and a sigmoid curve (four - parameter logistic curve) was constructed. The maximal forskolin -induced stimulated conversion is taken as maximum value and the maximal inhibition (usually at drug concentrations 10⁻⁶ M or 10⁻⁵ M) as minimum and these values were fixed during the fitting process. Thus, concentrations of the compound, causing 50% of the maximally obtained inhibition of forskolin-induced cAMP accumulation (EC₅₀), are averaged over several experiments and presented as mean pEC₅₀ ± SEM. Antagonist potency is assessed by co-incubating cells with a fixed agonist concentration and specified antagonist concentrations. Curve fitting procedures are identical to those used for estimating EC₅₀ values. Thus IC₅₀ values, i.e. the concentration that is able to achieve 50% of maximal antagonism that can be achieved by this compound. IC₅₀ values are corrected using a Cheng-Prussoff equation, correcting it for agonist concentration and EC₅₀ values that is obtained in the same experiment. Thus, K_{b} = IC₅₀ / (1+ [agonist]/EC₅₀, agonist).The corresponding pA₂ value is -log (K_{b}). Concentration-response curve fitting allows estimation of pEC₅₀ values and of maximal achievable effect (intrinsic activity or efficacy (ε). A full receptor agonist has ε = 1, a full receptor antagonist has ε = 0, and a partial receptor agonist has an intermediate intrinsic activity.

### DOSAGES

The affinity of the compounds of the invention for dopamine -D₂ receptors and serotonine reuptake sites was determined as described above. From the binding affinity measured for a given compound of formula (1), one can estimate a theoretical lowest effective dose. At a concentration of the compound equal to twice the measured Kᵢ-value, 100% of the receptors likely will be occupied by the compound. Converting that concentration to mg of compound per kg of patient yields a theoretical lowest effective dose, assuming ideal bioavailability. Pharmacokinetic, pharmacodynamic, and other considerations may alter the dose actually administered to a higher or lower value. The dosage expediently administered is 0.001 - 1000 mg/kg, preferably 0.1-100 mg/kg of patient's bodyweight.

### TREATMENT

The term 'treatment' as used herein refers to any treatme nt of a mammalian, preferably human condition or disease, and includes: (1) preventing the disease or condition from occurring in a subject which may be predisposed to the disease but has not yet been diagnosed as having it, (2) inhibiting the disease or condition, i.e., arresting its development, (3) relieving the disease or condition, i.e., causing regression of the condition, or (4) relieving the conditions caused by the disease, i.e., stopping the symptoms of the disease.

The preparation of the compounds having formula (I) will now be described in more detail in the following Examples.

### EXAMPLES

The H-atom of the N-H moiety of amines **I**-H to **V**-H can be replaced by **Q** in two different chemical ways, A and B, eventually leading to the compounds of the invention which are listed in Table 1 (*see below)*.

### Method A:

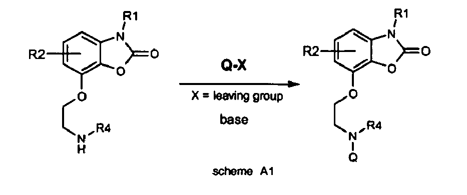

The compounds were prepared via the synthesis depicted in scheme A1: an amine (one of the five **I**-H to **V**-H, structures given below) was reacted with **Q**-X (X = leaving group like *e*.*g*. Cl, Br, I) in *e*.*g*. acetonitrile or butyronitrile with Et(*i*-Pr)₂N acting as a base, in some cases KI (or Nal) was added. Et₃N can be used instead of Et(*i*-Pr)₂N.

### Example 1:

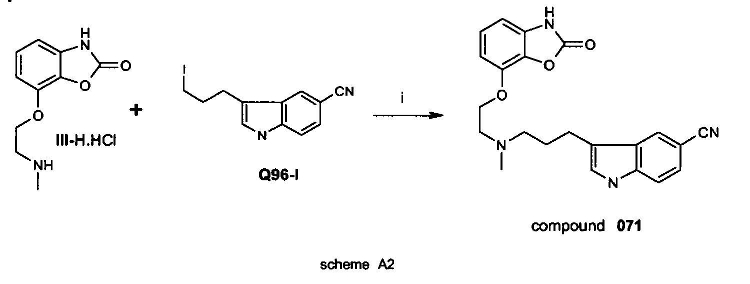

### Scheme A2, step i:

A mixture of 0.8 g. (3.27 mmol) amine **III**-H.HCl, 1.13 g (3.65 mmol) iodide and 2.31 ml (12.7mmol) diisopropylethylamine in 140 ml of butyronitril was heated at reflux temperature for 22 hours. The reaction mixture was allowed to reach room temperature and after concentration *in vacuo,* the residu was purified by flash column chromatography (SiO₂, eluent: ethylacetate /methanol/ammonia 92/7.5/0.5 v/v/v). The product containing fractions were concentrated *in vacuo* and the residue was suspended and stirred in diisopropylether. The resulting solid was isolated by filtration.Yield 0.71 g (55 %). M.p. 62-5 °C.

### Method B:

The compounds were prepared via the synthesis depicted in scheme B1: an amine (one of the five **I**-H to **V**-H, structures given below) was alkylated by means of a reductive alkylation. **Q**-OH was oxidized to the corresponding aldehyde **Q**'-CHO after which the reductive alkylation was performed. THF and DCE are suitable solvents for this type of reaction.

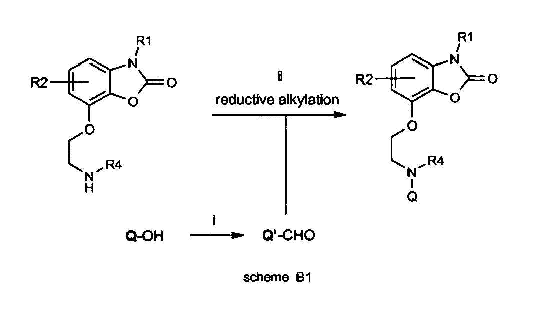

### Example 2:

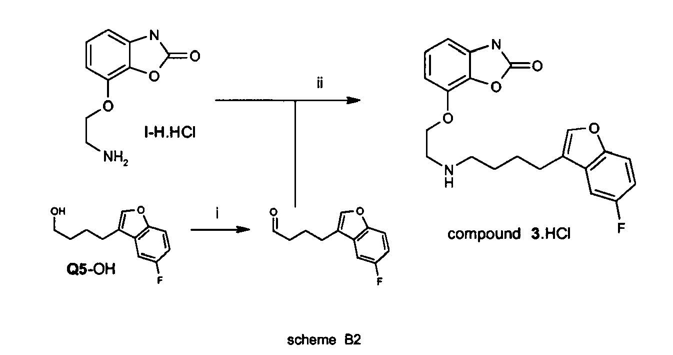

### Scheme B2, step i:

To a solution of 0.28 ml (3.3 mmol) of oxalylchloride in 7.5 ml of methylenechloride at -70 °C was added dropwise 0.47 ml (6.6 mmol) of DMSO. After 5 minutes, a solution of 0.58 g of the alcohol **Q5**-OH in 3 ml methylenechloride, was added dropwise and stirring was continued for another 20 minutes. Subsequently, 2.1 ml (15 mmol) of triethylamine were added and the temperature brought to room temperature. The reaction mixture was washed with 1% HCl (aq.), water, 5 % NaHCO₃, saturated NaCl and dried (Na₂SO₄). After removal of the drying agent by filtration and the solvent by concentration *in vacuo,* 0.54 g (92 %) of a yellow oil, containing the corresponding aldehyde of **Q5**-OH, was isolated.

### Scheme B2, step ii:

To a suspension of 0.54 g (2.34 mmol) of the aminehydrochlo ride **I**.HCl in 8 ml of MeOH was added 0.61 ml (3.51 mmol) DIPEA, molecular sieves (3 A) and a solution of 0.54 g (2.8 mmol) of the aldehyde (obtained in step i) in 4 ml MeOH.The resulting mixture was stirred overnight.
Then 0.14 g of NaBH₄ was added and after 1 hour of stirring the reaction was quenched with water. After extraction with ethylacetate, the combined organic fractions were dried on MgSO₄. After removal of the drying agent by filtration and the solvent by concentration *in vacuo*, the residu was subjected to flash column chromatography (SiO2, eluent: CH₂Cl₂/MeOH/NH₄OH 960/37.5/2.5), yielding the product which was converted to its HCl salt, **3**.HCl. Yield 0.16 g (18%). M.p.: 197-200 °C.

**Table 1: examples of compounds of the invention.**

| | | | | | | |
|---|---|---|---|---|---|---|
| Structures of amines (Z) and groups 'Q' (=T-Ar) are given below. In the column 'method', the general method (A or B) is given, and in case of method A, the next column gives the leaving group. | | | | | | |

| **Comp.** | **amine Z** | **Group Q** | **meth.** | **L-group** | **salt** | **melting r. °C** |
|---|---|---|---|---|---|---|
| | | | | | | |
| **1** | **I** | 2 | A | I | HCl | 146-148 |
| **2** | **I** | 3 | A | I | HCl | 201-204 |
| **3** | **I** | 5 | B | | HCl | 197-200 |
| **4** | **I** | 6 | A | I | HCl | 213.5-216 |
| **5** | **I** | 8 | A | I | HCl | 169-171 |
| **6** | **I** | 9 | A | I | HCl | 230-233 |
| **7** | **I** | 11 | A | I | HCl | 192-196 |
| **8** | **I** | 12 | A | I | HCl | 223-228 |
| **9** | **I** | 68 | A | Br | HCl | 194-196 |
| **10** | **I** | 69 | A | Br | HCl | 204-206 |
| **11** | **I** | 70 | A | Br | HCl | 179-181 |
| **12** | **I** | 71 | A | Br | free base | 153-155 |
| **13** | **I** | 72 | A | Br | free base | 124-125 |
| **14** | **I** | 73 | A | Br | HCl | 181-183 |
| **15** | **I** | 74 | A | Br | free base | Rf 0.36 (E1)* |
| **16** | **I** | 75 | A | Br | free base | Rf 0.08 (E1)* |
| **17** | **I** | 77 | A | Br | HCl | 211-215 |
| **18** | **I** | 78 | A | Br | HCl | 228-231 |
| **19** | **I** | 79 | A | I | HCl | 204-205 |
| **20** | **I** | 80 | A | I | HCl | 242-243 |
| **21** | **I** | 82 | A | I | HCl | 188-189 |
| **22** | **I** | 83 | A | I | HCl | 184-186 |
| **23** | **I** | 85 | A | Br | HCl | 212-214 |
| **24** | **I** | 86 | A | Br | HCl | 174-178 |
| **25** | **I** | 87 | A | I | HCl | 177-179 |
| **26** | **I** | 88 | A | I | HCl | 185-187 |
| **27** | **I** | 89 | A | I | HCl | 221-223 |
| **28** | **I** | 90 | A | I | HCl | 204-206 |
| **29** | **I** | 94 | A | I | HCl | 180-185 |
| **30** | **I** | 96 | A | I | HCl | 234-238 |
| **31** | **I** | 97 | A | I | HCl | 175-177 |
| **32** | **II** | 94 | A | I | HCl | 194-198 |
| **33** | **III** | 2 | A | I | HCl | 136-138 |
| **34** | **III** | 3 | A | I | HCl | 259-262 |
| **35** | **III** | 5 | A | I | HCl | 167-170 |
| **36** | **III** | 6 | A | I | HCl | 207-209.5 |
| **37** | **III** | 7 | A | I | HCl | 219-223 |
| **38** | **III** | 8 | A | I | HCl | 147-149 |
| **39** | **III** | 9 | A | I | HCl | 247-250 |
| **40** | **III** | 10 | A | I | HCl | 110-118 |
| **41** | **III** | 11 | A | I | free base | 103-105 |
| **42** | **III** | 12 | A | I | free base | 99-105 |
| **43** | **III** | 18 | A | I | HCl | 215-218 |
| **44** | **III** | 38 | A | I | HCl | 181-184 |
| **45** | **III** | 41 | A | Br | HCl | 167-170 |
| **46** | **III** | 68 | A | Br | HCl | 217-219 |
| **47** | **III** | 69 | A | Br | HCl | 169-171 |
| **48** | **III** | 70 | A | Br | HCl | 167-169 |
| **49** | **III** | 71 | A | Br | free base | Rf 0.21 (EtOAc) |
| **50** | **III** | 72 | A | Br | free base | Rf 0.24 (E1)* |
| **51** | **III** | 73 | A | Br | HCl | 163-165 |
| **52** | **III** | 74 | A | Br | free base | Rf 0.48 (E1)* |
| **53** | **III** | 75 | A | Br | free base | 82-83 |
| **54** | **III** | 77 | A | Br | HCl | 144-146 |
| **55** | **III** | 78 | A | Br | HCl | 162-165 |
| **56** | **III** | 79 | A | I | HCl | 185-186 |
| **57** | **III** | 80 | A | I | HCl | 170-171 |
| **58** | **III** | 82 | A | I | HCl | 164-165 |
| **59** | **III** | 83 | A | I | HCl | 168-170 |
| **60** | **III** | 84 | A | I | HCl | 188-190 |
| **61** | **III** | 85 | A | Br | HCl | 115-118 |
| **62** | **III** | 86 | A | Br | HCl | 102-108 |
| **63** | **III** | 87 | A | I | HCl | 155-157 |
| **64** | **III** | 88 | A | I | HCl | 167-169 |
| **65** | **III** | 89 | A | I | HCl | 177-179 |
| **66** | **III** | 90 | A | I | HCl | 132-134 |
| **67** | **III** | 91 | A | Br | HCl | 204-207 |
| **68** | **III** | 92 | A | Br | HCl | 169-172 |
| **69** | **III** | 93 | A | Br | HCl | 152-156 |
| **70** | **III** | 94 | A | I | HCl | 198-202 |
| **71** | **III** | 96 | A | I | free base | 162-165 |
| **72** | **III** | 97 | A | I | HCl | 172-173 |
| **73** | **III** | 98 | A | I | HCl | 239-242 |
| **74** | **IV** | 1 | A | I | HCl | 203-207 |
| **75** | **IV** | 3 | A | I | HCl | 215-218 |
| **76** | **IV** | 4 | A | I | HCl | 173-176.5 |
| **77** | **IV** | 5 | A | I | HCl | 184.5-188 |
| **78** | **IV** | 6 | A | I | HCl | 188-195 |
| **79** | **IV** | 7 | A | I | HCl | 216-219 |
| **80** | **IV** | 8 | A | I | HCl | 190-194 |
| **81** | **IV** | 9 | A | I | HCl | 229-233 |
| **82** | **IV** | 11 | A | I | HCl | 209-213 |
| **83** | **IV** | 12 | A | I | HCl | 113-116 |
| **84** | **IV** | 19 | A | I | HCl | 182-190 |
| **85** | **IV** | 20 | A | I | HCl | 175-177 |
| **86** | **IV** | 21 | A | I | HCl | 202-204 |
| **87** | **IV** | 22 | A | I | HCl | 186-188 |
| **88** | **IV** | 23 | A | I | HCl | 205-207 |
| **89** | **IV** | 24 | A | I | HCl | 175-177 |
| **90** | **IV** | 38 | A | I | HCl | 149-154 |
| **91** | **IV** | 41 | A | Br | HCl | 154-156 |
| **92** | **IV** | 51 | A | I | HCl | 199-202 |
| **93** | **IV** | 72 | A | Br | HCl | 194-198 |
| **94** | **IV** | 73 | A | Br | HCl | 139-143 |
| **95** | **IV** | 75 | A | Br | HCl | 161.5-163.5 |
| **96** | **IV** | 76 | A | Br | HCl | 139.5-140.5 |
| **97** | **IV** | 94 | A | I | HCl | 175-180 |
| **98** | **IV** | 96 | A | I | HCl | 179-181 |
| **99** | **IV** | 97 | A | I | HCl | 209-212 |
| **100** | **IV** | 98 | A | I | HCl | 168-172 |
| **101** | **V** | 3 | A | I | HCl | 207-208 |
| **102** | **V** | 5 | A | I | HCl | 190-192 |
| **103** | **V** | 6 | A | I | HCl | 191-193 |
| **104** | **V** | 9 | A | I | HCl | 219-223 |
| **105** | **V** | 11 | A | I | HCl | 208-212 |
| **106** | **V** | 57 | A | Br | HCl | 170-174 |
| **107** | **V** | 94 | A | I | HCl | 161-164 |
| **108** | **V** | 96 | A | I | free base | 60-75 |

| | | | | | | |
|---|---|---|---|---|---|---|
| *(E1)** *= EtOAc*/*MeOH 9*/*1* | | | | | | |

The aryloxyethylamines, 'Z' in formula (1), used in these methods are indicated as **I**- H to **V**-H, wherein the dot on the N-atom is the attachment point for 'T-Ar' of formula (1) :

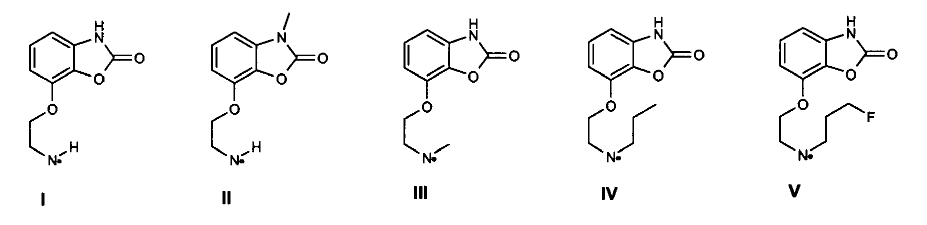

### Synthesis of amine I-H:

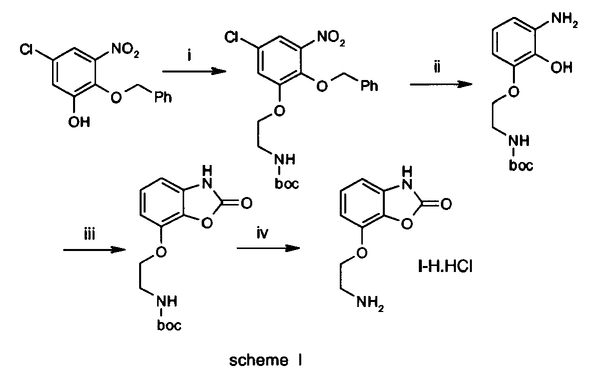

### Scheme I, steps i,ii, iii and iv:

The steps i, ii, iii and iv respectively, were analogously performed to the steps vi, vii, viii and ix in scheme III respectively.

### Synthesis of amine II-H:

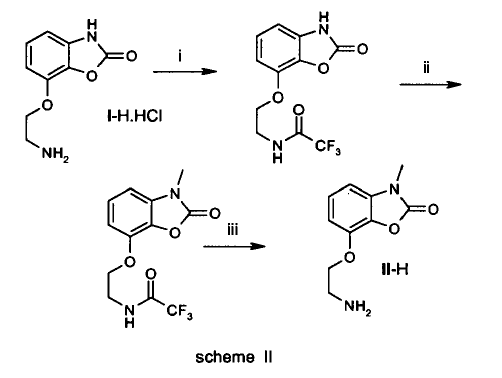

### Scheme II, step i:

2.5 g (10.85 mmol) was suspended in 125 ml of DCM to which 4.15 ml (23.85 mmol) of DIPEA were added, stirring was continued for 15 minutes. While cooling (ice bath) 1.7 ml (11.95 mmol) of trifluoroacetic acid anhydride in 10 ml of DCM were added dropwise. The ice bath was removed and stirring continued for 14 hours. The reaction mixture was concentrated in vacuo, the residue subjected to flash column chromatography (SiO₂, eluent DCM/MeOH 98/2) which yielded 2.76 g (88%) of a solid containing the trifluoroacetylated derivative.

### Scheme II, step ii:

2.48 g (8.55 mmol) of the trifluoroacetylated derivative (from step i), was dissolved in 125 ml of acetone after which 3.9 g (28.2 mmol) of potassium carbonate and 0.59 ml (9.4 mmol) of methyl iodide were added. After 14 hours, the reaction mixture was concentrated in vacuo, the residue subjected to flash column chromatography (SiO ₂, eluent DCM/MeOH 99/1) which yielded 2.76 g (88%) of a solid containing the corresponding N-methylated compound.

### Scheme II, step iii:

2.25 g (7.4 mmol) of the N-methylated compound (from step ii) and 7.4 g (88 mmol) of sodium hydrogencarbonate were taken up in a mixture of 90 ml of water and 35 ml of methanol. Under a nitrogen atmosphere, the reaction mixture was stirred and refluxed for 14 hours. The reaction mixture was concentrated in vacuo, the residue subjected to flash column chromatography (SiO ₂, eluent DMA 0.50) which yielded 1.34 g (87%) of a solid containing the amine **II-**H as a free base.

### Synthesis of amine III-H:

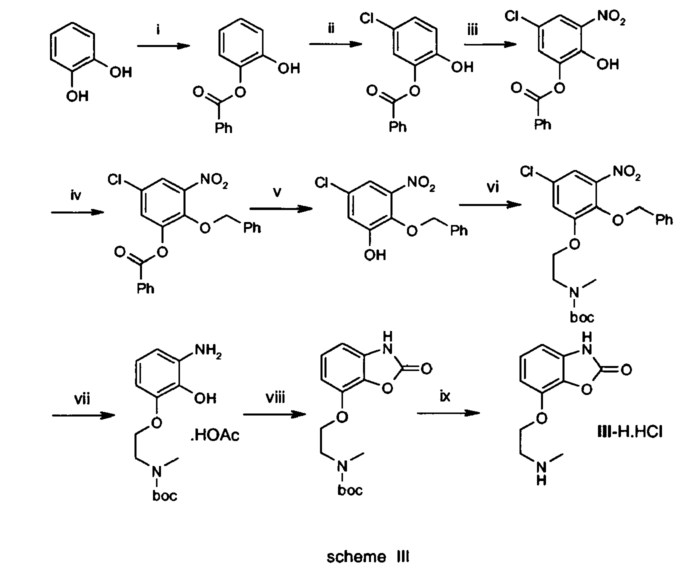

### Scheme III, step i:

Reaction was carried out under a nitrogen atmosphere and with a mechanical stirrer. 99.2 g (900 mmol) of catechol was dissolved in 450 ml of 2M NaOH to which 7.4 g (41 mmol) of Na₂S₂O₅ were added. A solution of 133.3 g (110.1 ml) of benzoyl chloride in 125 ml of toluene was added in 20 minutes dropwise to the catechol solution. After 2.5 hours the reaction mixture was brought to room temperature and 200 ml of petroleum ether were added. Stirring was continued for 30 minutes . The precipitate was filtered and the residu washed with water (2x), after which it was allowed to air-dry for 45 minutes . Then the residu was solved in ref luxing toluene (a small amount of water which formed was removed), the resulting solution filtered. To the filtrate 900 ml of petroleum ether was added. After 48 hours the precipitate was collected by filtration, yielding 139 g (72%) of benzoylated catechol.

### Scheme III, step ii:

139 g (650 mmol) of the benzoylated catechol (from step i) was taken up in 450 ml of DCM, after which 1 g of AlCl₃ and 10 drops of Ph₂S were added. At 10-15 °C, a solution of 58 ml of SO₂Cl₂ in 120 ml of DCM was added dropwise to the reaction mixture. The mixture was allowed to react further at room temperature for 14 hours. The precipitate was washed (3x) with DCM and yielded 130.1 g (81%) of pure chlorinated product.

### Scheme III, step iii:

130 g (523 mmol) of the chlorinated product (from step ii) was taken up in 1000 ml of acetic acid, the temperature was brought to 15 °C. Subsequently, a solution of 34 ml of fuming nitric acid in 150 ml of acetic acid was added dropwise to the reaction mixture. After about half of the nitric acid solution had been added, the reaction mixture turned red. Stirring at room temperature was continued for 14 hours. The mixture was poured into 3000 ml of ice/water. The suspension which formed was filtered and washed three times with water, after which the residu was dried. Chromatography (SiO₂, eluent: DCM) yielded 124 g (81%) of the pure nitrated product.

### Scheme III, step iv:

124 g (422 mmol) of the nitrated product (from step iii) was taken up in 900 ml of dry acetone. Subsequently, 74 ml of benzylbromide, 125 g magnesium sulfate hydrate and 9.25 g of sodium iodide were added. The mixture was brought to reflux temperature after which 60 g of powdered potassium carbonate was added portionwise. To keep the mixture stirrable, 300 ml of acetone were added. Refluxing was continued for 14 hours after which the reaction mixture was cooled and concentrated in vacuo. The residu was taken up in DCM and water, the whole was filtered over hyflo. The layers were separated, the water fraction was extracted with DCM (2x), the combined organic fractions dried on sodium sulfate. After removal of the drying agent and solvent (in vacuo), the emerging solid was washed (5x) with petroleum ether. 134 g (83%) of the benzylated product was isolated.

### Scheme III, step v:

Under a nitrogen atmosphere, 134 g (350 mmol) of the benzylated product (from step iv) was taken up in 1000 ml of ethanol. While stirring, a solution of 40 g of potassium hydroxide in 300 ml of ethanol, was added dropwise to the reaction mixture. When addition was complete, the mixture was allowed to reflux for 6 hours. The cooled reaction mixture was filtered, the residu washed with ethanol, the filtrate was concentrated in vacuo. The concentrate was taken up in water and diethylether, the water layer extracted again with diethylether. The combined organic layers were washed with brine and subsequently concentrated in vacuo. The residu was chromatographed twice (SiO₂, DCM), yielding almost pure product. The latter was stirred in petroleum ether, after which a solid could be isolated (76.4g, 78%) containing the pure phenol.

### Scheme III, step vi:

While stirring and under a nitrogen atmosphere, 20 g (71.6 mmol) of the phenol (from step v), 24 g triphenylphosphine and 20 g of N -Boc-N-methyl aminoethanol (see Basel et al., J. Org. Chem., 65(2000)6368 ) were added to 500 ml of toluene, after which the mixture was brought to 0 °C. To the latter, a solution of 28.6 ml DIAD in 100 ml of toluene was added dropwise in 30 minutes. After one hour, stirring was continued at room temperature for 14 hours. The reaction mixture was concentrated in vacuo, the residu treated with diethylether/petroleum ether, which caused the PPh₃O to precipitate. The precipitate was filtered, the residu washed with diethylether, the filtrate was concentrated in vacuo. The concentrate was chromatographed twice: (SiO₂, eluent: DCM) to remove the DIADH₂, secondly with (SiO₂, eluent: diethylether/petroleum ether ½). 31 g (100%) was isolated of the Mitsunobu product.

### Scheme III, step vii:

31 g (71 mmol) of the Mitsunobu product (from step vi) was taken up in 210 ml of water and 30 ml of acetic acid. 0.5 g of Pd(OH)₂ /C were added and hydrogenation was started for 14 hours at one atmosphere. Subsequently the reaction mixture was filtered over hyflo, the latter thoroughly washed with ethanol, the filtrate concentrated in vacuo. The concentrate was taken up in ethanol and concentrated again (2x), a third time with DIPE. 23.7 g (98%) of a residu was left containing the aminophenol.

### Scheme III, step viii:

23.7 g (69.3 mmol) of the amino phenol (from step vii) and 38 g (236 mmol) of CDI were added to 800 ml of dry THF. Subsequently 125 ml of DIPEA were added after which the mixture was refluxed for 14 hours. First chromatography (SiO ₂, eluent: ethylacetate) removed the imidazole, second chromatography (SiO₂, eluent: first diethylether/petroleum ether 1/1, then 2/1, then diethylether, then ethylacetate) yielded after concentration in vacuo 18 g of residu. The latter was stirred in DIPE after which it was filtered, the residu containing pure benzoxazolone (15.8 g (74%)).

### Scheme III, step ix:

14.97 g (48.6 mmol) of the Boc protected compound obtained in step viii were suspended in 500 ml of 1 M ethanolic HCl and the mixture was stirred at 55 °C for 1 hour. The reaction mixture was concentrated *in vacuo* and the residue was suspended in diisopropylether. The resulting solid was filtered, yielding 11.71 g (99 %) of the desired product, **III**-H.HCl.

### Synthesis of amine IV-H:

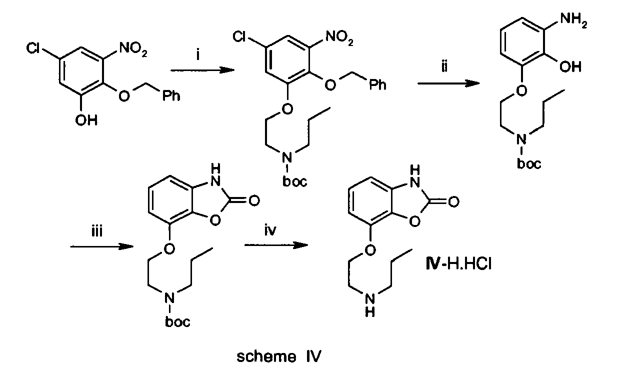

### Scheme IV, steps i,ii, iii and iv:

The steps i, ii, iii and iv were analogously performed to the steps vi, vii, viii and ix in scheme III respectively.

### Synthesis of amine V-H:

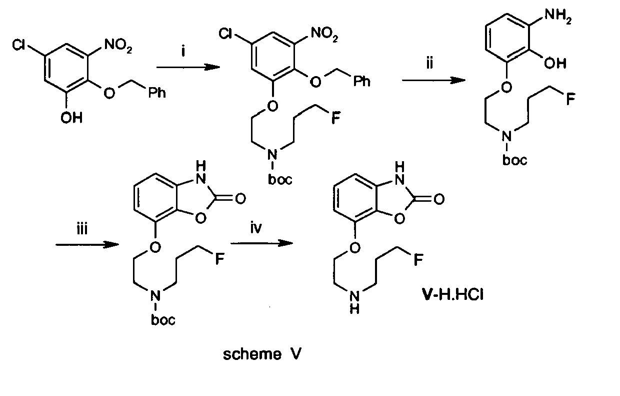

### Scheme V, steps i,ii, iii and iv:

The steps i, ii, iii and iv were analogously performed to the steps vi, vii, viii and ix in scheme III respectively.

Below, the various forms **Q1** to **Q104** are given: **Q. :**

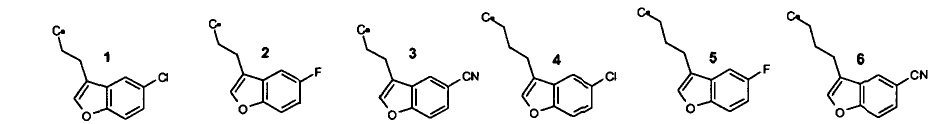

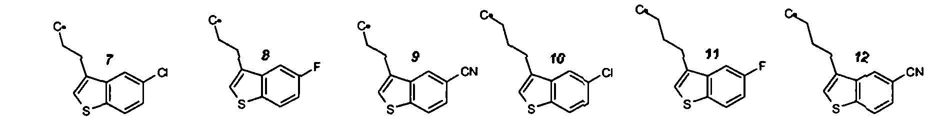

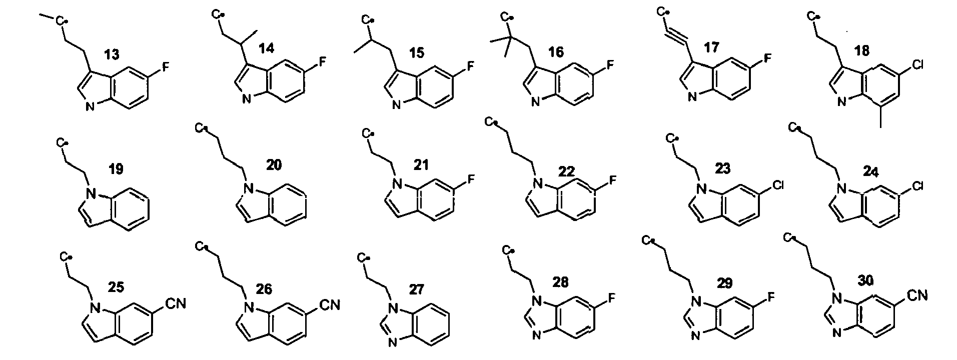

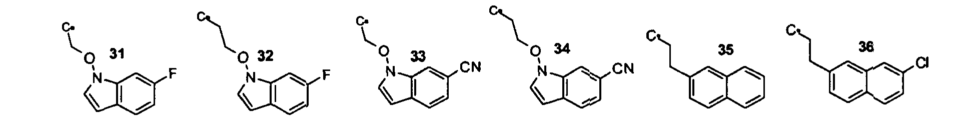

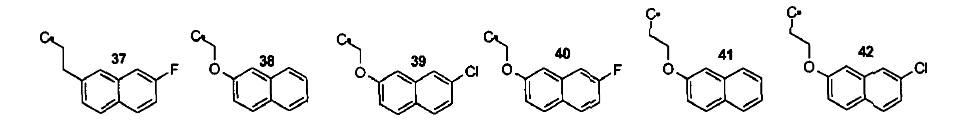

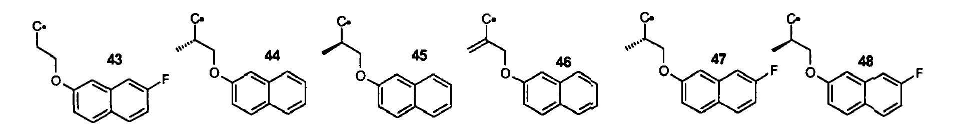

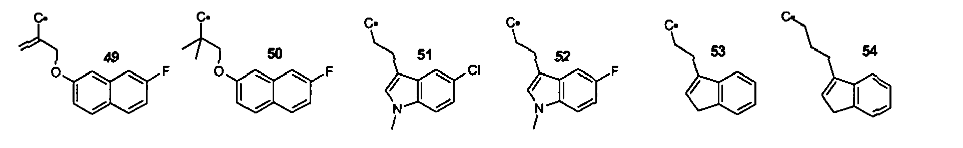

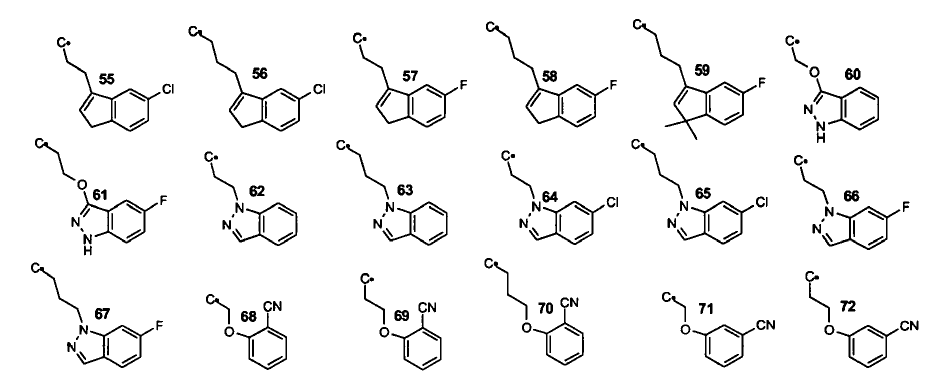

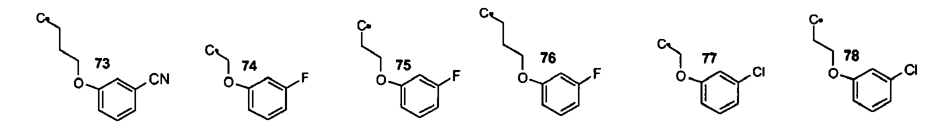

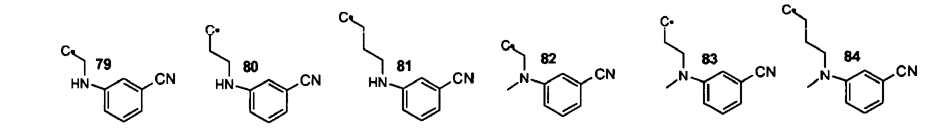

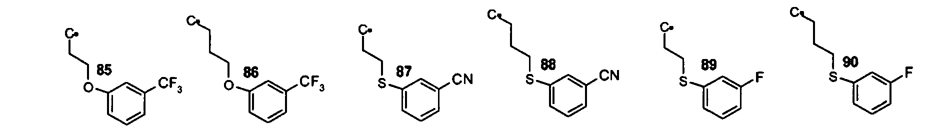

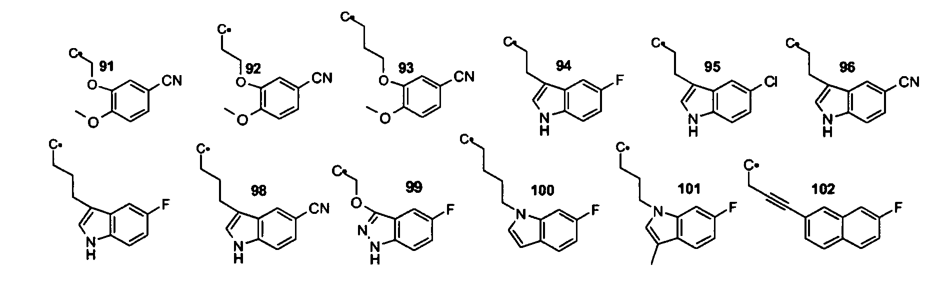

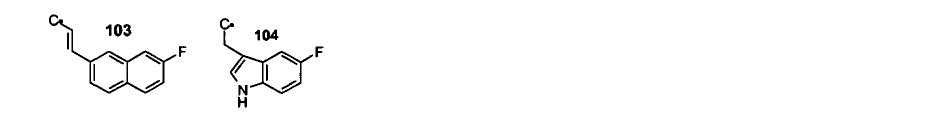

In these formulae 'Q', corresponding to 'T-Ar' in formula (1), the dot represents the attachments to the fragment 'Z'.

### Synthesis of Q1-6:

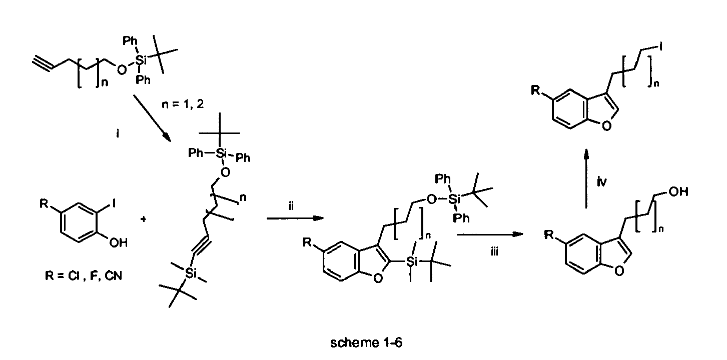

All starting materials (phenols and alkynes) were prepared according to procedures described in the literature:
Alkynes: Davison, Edwin C.; Fox, Martin E.; J. Chem. Soc. Perkin Trans. 1 ; 12(2002) 1494-1514. Yu, Ming; Alonso-Alicia, M.; Bioorg. Med. Chem.; 11 (2003)2802-2822.
Phenols: Buchan; McCombie; J. Chem. Soc.; 137 (1931) 144. Finger et al; J. Amer. Chem. Soc.; 81 (1959) 94, 95, 97. Berg; Newbery; J. Chem. Soc.; (1949) 642-645.

### Scheme 1-6, step i:

### R=CN, n=2

A stirred solution of the silylated alcohol (3.35 g, 10 mmol) in 20 ml of dry THF was cooled to -70 °C. 2.5M n-BuLi (4.8 ml, 12 mmol) was slowly added dropwise at such a rate that the temperature was kept below -65 °C. The solution was allowed to warm to -20 °C and stirring was continued for 1 hour during which the color of the solution changed from light to dark yellow. The solution is again cooled to -70 °C and a solution of tert-butyldimethylsilylchloride (1.66 g, 11 mmol) in 15 ml of dry THF is slowly added dropwise in 10 minutes. The reaction mixture was allowed to warm to room temperature and stirring was continued for 20 h. The reaction mixture was quenched by the addition of saturated NH₄Cl and extracted 2x with Et₂O. The combined Et₂O layers were washed with 5% NaHCO₃ (1x) and H₂O (1x) and dried (Na₂SO₄). The Et₂O fraction was concentrated under reduced pressure and the residue was chromatographed (SiO₂) using DMA/petroleum ether 1/5 as eluent to give 3.35 g (75%) of the silylated alkyne as a colorless oil.

### Scheme 1-6, step ii:

A mixture of 4-cyano-2-iodophenol (1.23 g, 5 mmol), silylated alkyne (from step i) (2.18 g, 5 mmol), LiCl (0.21 g, 5 mmol) and Na₂CO₃ (2.38 g, 22.5 mmol) in 20 ml DMF was degassed by bubbling nitrogen through the solution for 2 h. Pd(OAc)₂ (50 mg, 0.20 mmol) was added and the reaction mixture was stirred for 7 hours at 100 °C. H₂O and hexane were added and the mixture was filtered over hyflo. After separation of the hexane layer, the aqueous layer was extracted with hexane (1x). The combined hexane layers were washed with H₂O (1x) and brine (1x). The hexane fraction was partially evaporated under reduced pressure and 8 g of silicagel was added and stirring was continued for 15 minutes The silica is filtered off and the filtrate is concentrated under reduced pressure. The residue was chromatographed (SiO₂) using Et₂O/petroleum ether 1/9 as the eluent to give 0.93 g (35%) of the benzfurane derivative as a light yellow oil.

### Scheme 1-6, step iii:

This step was done analogously to step iv in scheme 79-84-b.

### Scheme 1-6, step iv:

This step was done analogously to step iii in scheme 79-84.

### Synthesis of Q7-9:

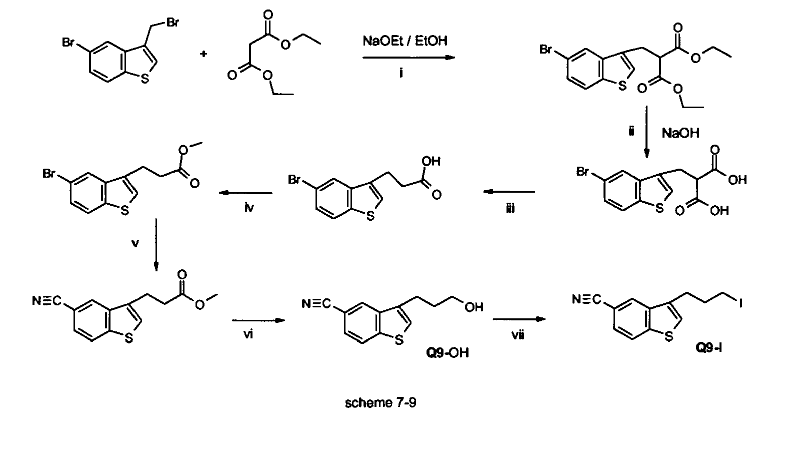

The 5-bromobenzthiophene was prepared according to: Leclerc, V.; Beaurain, N.; Pharm. Pharmacol. Commun., 6(2000)61-66.

### Scheme 7-9, step i:

Sodium metal (4.5 g, 195.9 mmol) was added in pieces to 260 ml of absolute EtOH. The malonic ester (116 ml, 779 mmol) was added and the reaction mixture was stirred under a nitrogen atmosphe re for 30 minutes . The 5-bromobenzthiophene (29.5 g, 97.2 mmol) was added as a suspension in 125 ml of absolute EtOH and stirring was continued at reflux for 18 h. The solvent was evaporated under reduced pressure after which 250 ml H₂O and 15 g NH₄Cl were added to the residue. The aqueous layer was extracted with CH₂Cl₂ (2x) and the combined organic layers were dried (Water Repelling Filter) and the filtrate concentrated in vacuo (by means of an oil pump, 8 mbar). The residue was chromatographed (SiO₂) with CH₂Cl₂/petroleumether 3/2 to give 23.9 g (64%) of the di-ester.

### Scheme 7-9, step ii:

This step was carried out analogous to step ii from scheme 59.

### Scheme 7-9, step ii:

This step was carried out analogous to step iii from scheme 59.

### Scheme 7-9, step iv:

This step was carried out analogous to step iii from scheme 10-12.

### Scheme 7-9, step v

This step was carried out analogous to step v from scheme 10-12.

### Scheme 7-9, step vi:

This step was carried out analogous to step iii from scheme 79-84.

Derivatives of **Q7** and **Q8** were prepared analogously to the above described procedures.

### Synthesis of Q10-12:

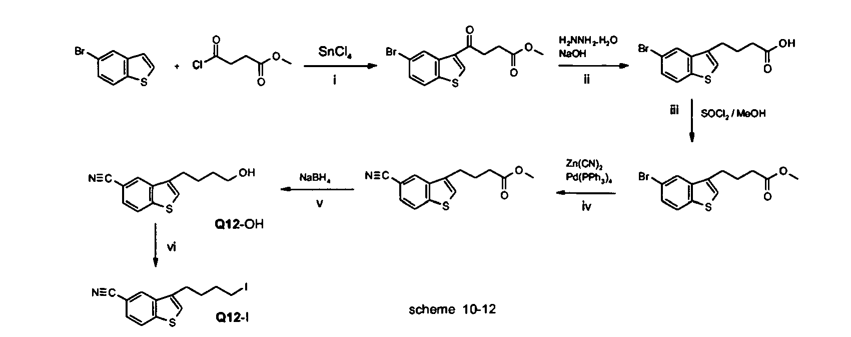

All reagents were commercially available. The 5 -bromobenzthiophene was prepared according to Badger et al., J. Chem. Soc., (1957) 2624, 2628.

### Scheme 10-12, step i:

To a stirred mixture of 5-bromobenzthiophene (22.5 g, 105.6 mmol) and the acid chloride (17.4 ml, 141.3 mmol) in 135 ml benzene at 0 °C, SnCl₄ (43.1 ml, 368 mmol) was added in 2 h. Stirring was continued for 4 hours at the s ame temperature. The reaction mixture was poured into a mixture of 95 ml concentrated HCl (36-38%) in ice. The reaction mixture was extracted with EtOAc and the organic layer was washed with H₂O (3x), 1N NaOH (1x), 5% NaHCO₃ and H₂O (2x). The EtOAc fraction was dried (MgSO₄). The drying agent was removed by filtration and the solvent by evaporation under reduced pressure. The residue was recrystallized from 950 ml MeOH and chromatographed with Et₂O/petroleum ether 1/1 as eluent to give 23.3 g (68%) of the acylated benzthiophene.

### Scheme 10-12, step ii:

To a stirred mixture of the acylated benzthiophene (23.3 g, 71.3 mmol) and powdered NaOH (23 g, 575 mmol) in 285 ml diethyleneglycol, hydrazine hydrate (23 ml, 474 mmol) was added. Stirring was continued for 2 hours at 145 °C after which additional stirring for 2 hours at 180 °C was needed to complete the conversion. The reaction mixture was poured onto ice and acidified with concentrated HCl (36 -38%). The aqueous layer was extracted with Et₂O and the organic layer was washed with H₂O (3x) and brine (1x) and dried (MgSO₄). The drying agent was removed by filtration and the solvent by evaporation under reduced pressure yielded 19.7 g (93%) of the acid.

### Scheme 10-12, step iii:

At -5 °C, 29 ml of thionyl chloride were added dropwise in 30 minutes to 250 ml of MeOH . The mixture was stirred for 15 minutes during which the temperature was kept between -10 °C and -5 °C. The acid (19.7 g, 65.9 mmol) was added in one time to the cooled solution. The reaction mixture was stirred for 1 hour after which is was allowed to warm to room temperature and stirred for an additional 20 h. The reaction mixture was concentrated in vacuo and the residue was chromatographed (SiO₂) with CH₂Cl₂ as the eluent to give 20.6 g (100%) of the methyl ester.

### Scheme 10-12, step iv:

A mixture of the methyl ester (20.6 g, 65.8 mmol) and zinc cyanide (4.64 g, 39.5 mmol) in 85 ml of dry DMF was degassed by bubbling nitrogen through the solution for 1 h. Palladium tetrakis, Pd(PPh₃)₄, (3.8 g, 3.29 mmol) was added under a nitrogen atmosphere and the reaction mixture was stirred for 16 hours at 90 °C. The reaction mixture was diluted with 200 ml toluene and filtered through a pad of Hyflo. The organic layer was washed with 5% NaHCO₃ (2x) and brine (1x). The organic layer was dried (MgSO₄). The drying agent was removed by filtration and the solvent by evaporation under reduced pressure. The residue was chromatographed (SiO₂) using CH₂Cl₂/petroleum ether 3/2 → CH₂Cl₂ as eluent to give 15.6 g (92%) of the 5-cyanobenzthiophene.

### Scheme 10-12, step v:

To a stirring solution of the 5-cyanobenzthiophene (15.6 g, 60.2 mmol) in 250 ml 96% EtOH at 15 °C was added sodium borohydride (22.8 g, 602 mmol) in one time. The reaction mixture was stirred at room temperature for 48 h. H₂O was added and the aqueous layer was extracted with Et₂O (3x). The combined organic layers were washed with brine (1x). The Et₂O fraction was dried (MgSO₄). The drying agent was removed by filtration and the solvent by evaporation under red uced pressure. The residue was chromatographed (SiO₂) with Et₂O/CH₂Cl₂ 1/9 as eluent to give 9.2 g (66%) of the alcohol **Q12**-OH.

### Scheme 10-12, step vi:

Was prepared according to the procedure described in Scheme 79 -84, step iii.

**Q10**-OH and **Q11**-OH were prepared similarly using steps i, ii, iii and v respectively.

### Synthesis of Q13:

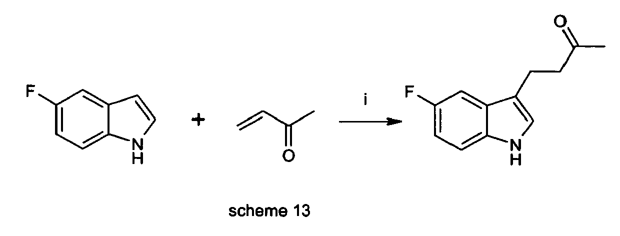

### Scheme 13, step i:

0.56 g (1.5 mmol) of CeCl₃.7H₂O and 0.22 g (1.5 mmol) of sodium iodide were together with 2.3 g of silica (SiO₂) taken up in 33 ml of acetonitrile. The resulting mixture was stirred for 14 hours. Then the mixture was concentrated in vacuo until a yellowish powder remained. Subsequently, 0.68 g (5 mmol) of 5-fluoroindole were added, then 0.35 g (5 mmol) of methylvinylketone were added, the solid mixture turned greyish after which the color returned again to yellow. After 4 hours the mixture was put on top of a flash chromatographic column (SiO₂) and eluted with DCM. 0.80 g (78%) of the indolylketone could be isolated.

### Synthesis of Q14:

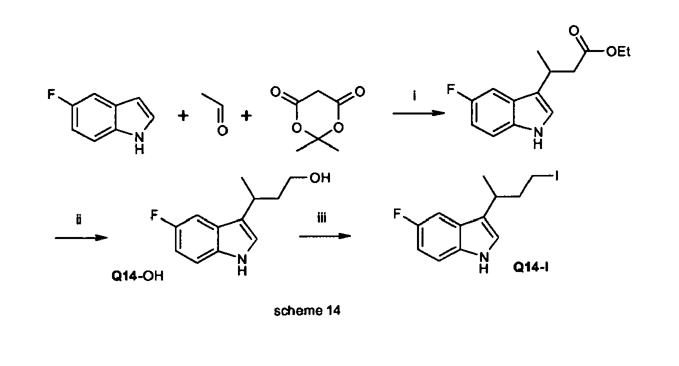

### Scheme 14, step i:

4.51 g (33.4 mmol) of 5-fluoroindole and 4.81 g (33.4 mmol) of Meldrum's acid were taken up in 40 ml of acetonitrile. Subsequently, 3.75 ml (66.8 mmol) of acetic aldehyde were added to the reaction mixture, after which stirring was continued for 24 hours. The reaction mixture was concentrated in vacuo, and dissolved again in 67 ml of pyridine after which 6.7 ml of absolute ethanol and 0.84 g of cupper powder were added. The mixture was brought to reflux for three hours. The reaction mixture was cooled and concentrated in vacuo, the residu was taken up in diethylether, the suspension filtered and the filtrate was washed with 1 M HCl, 20% NH₄Cl (H₂O) and water, respectively. The organic layer was dried (MgSO₄) and concentrated in vacuo, the residu purified by flash column chromatography (SiO₂, eluent: DCM/petroleum ether 4/1), yielding 6.43 g (77%) of the indolylalkylester.

### Scheme 14, step ii:

4 g (105.3 mmol) of LiAlH₄ were taken up in 100 ml of THF, after which 8.1 g (32.5 mmol) of indolylalkylester (from step i) dissolved in 50 ml of THF, were added dropwise during 30 minutes . The reaction mixture was brougt to reflux for 45 minutes . After cooling (ice bath) a mixture of 4 ml of water in 10 ml of THF, 8 ml of 2M NaOH, and 8 ml of water were added dropwise to the reaction mixture respectively. The latter mixture was brought to reflux again for 30 minutes . After cooling the reaction mixture was filtered and the filtrate concentrated in vacuo, the residu purified by flash column chromatography (SiO₂, eluent: diethylether), yielding 6.73 g (100%) of the pure indolylalkylalcohol **Q14**-OH.

### Scheme 14, step iii:

The conversion of the resulting alcohols to the corresponding iodo derivatives was performed according to the procedure described in scheme 79 -84 step iii.

### Synthesis of Q15:

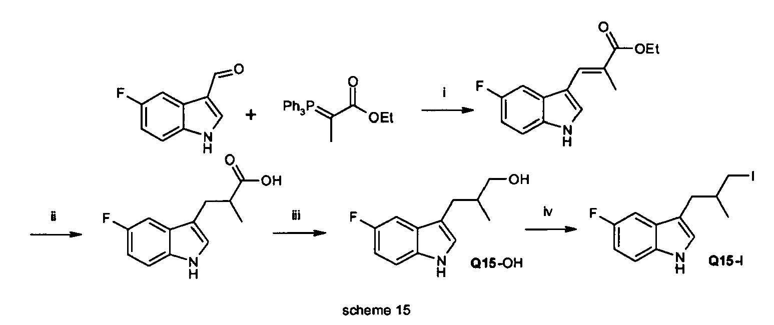

### Scheme 15, step i:

5.5 g (33.7 mmol) of 5-fluoro-3-carbaldehyde and 18.3 g (50.6 mmol) of the triphenylphosphine derivative were taken up in 165 ml of dioxane, after which th e mixture was brought to reflux for 3 hours. After cooiing, the reaction mixture was concentrated in vacuo and the residu purified by flash column chromatography (SiO₂, eluent: DCM), yielding 8.72 g (100%) of the pure indolylalkenylester.

### Scheme 15, step ii:

7.49 g (30.3 mmol) of the (from step i) were dissolved in 200 ml of absolute ethanol and 0.75 g of 10% Pd/C were added after which hydrogenation was started at room temperature and 1 atmosphere. After 14 hours the mixture was filtered over hyflo, the filtrate concentrated in vacuo, yielding 7.54 g (100%) of the corresponding indolylalkylester.

### Scheme 15, step iii:

3.7 g (98.2 mmol) LiAlH₄ was taken up in 100 ml of dry THF after which a solution 7.54 g (30.3 mmol) of the indolylalkylester (of step iii) in 50 ml of dry THF was added dropwise to the reaction mixture in 30 minutes . After cooling (ice bath) a mixture of 3.7 ml of water in 10 ml of THF, 7.4 ml of 2M NaOH, and 7.4 ml of water were added dropwise to the reaction mixture respectively. The lat ter mixture was brought to reflux again for 30 minutes . After cooling the reaction mixture was filtered and the filtrate concentrated in vacuo, the residu purified by flash column chromatography (SiO₂, eluent: diethylether), yielding 6.27 g (100%) of the pure indolylalkylalcohol **Q15**-OH.

### Scheme 15, step iv:

The conversion of the resulting alcohols to the corresponding iodo derivatives was performed according to the procedure described in scheme 79 -84 step iii.

### Synthesis of Q16:

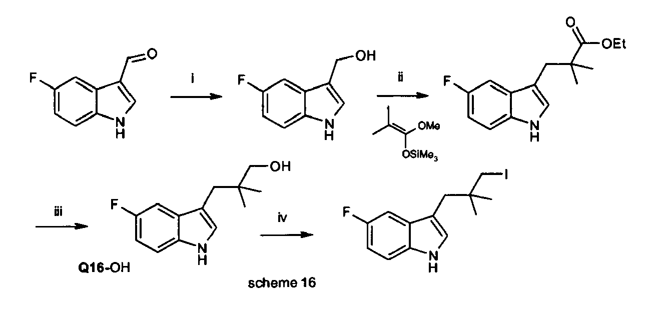

### Scheme 16, step i:

4.89 g (30 mmol) of 5-fluoro-3-carbaldehyde were solved in 100 ml of methanol and the solution cooled in an ice bath. 3.42 g (90 mmol) of NaBH₄ were added portionwise in 15 minutes time. After 30 minutes the ice bath was removed after which the reaction was stirred for another 30 minutes . 400 ml of water were added after which extraction with DCM took place (4x), the collected organic fractions were filtered over a water repellant filter, the dry filtrate carefully concentrated in vac uo (T< 25 °C), eventually yielding 4.95 g (100 %) of the corresponding indolylmethylalcohol, which was directly used in the next step.

### Scheme 16, step ii:

4.95 g (30 mmol) of the indolylmethylalcohol (from step i) were dissolved in 300 ml of DCM after which 12.2. ml (60 mmol) of 1,1 -dimethyl-2methoxy-2-trimethylsilyloxyethene, and 1.76 g (3 mmol) of Mg(NTf₂)₂ hydrate were added. The mixture was stirred for one hour. Subsequently the reaction mixture was washed with water, and the organic layer was filtered over a water repellant filter, the dry filtrate carefully concentrated in vacuo. The residu was purified by flash column chromatography (SiO₂, eluent: DCM), yielding 6.9 g (92%) of the corresponding pure indolylalkyl ester.

### Scheme 16, step iii:

This step was done analogous to Scheme 15, step iii.

### Scheme 16, step iv:

The conversion of the resulting alcohols to the corresponding iodo derivatives was performed according to the procedure described in scheme 79-84 step iii. The compound isolated was not the iodide, but the corresponding triphenylphosphonium iodide salt which can be tranformed into the desired iodide **Q16**-I by refluxing the salt in butyronitrile. After work up the the crude product was purified by flash column chromatography (SiO₂, eluent: DCM).

### Synthesis of Q17:

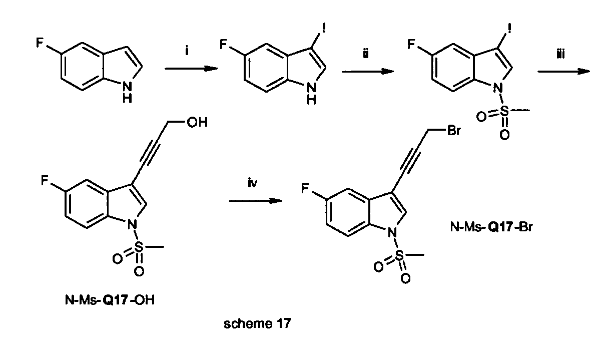

### Scheme 17, step i:

4.73 g (84.4 (mmol) of KOH were added to a cooled (water bath) solution of 3.0 g (22.2 mmol) of 5-fluoroindole in 11 ml of DMF. After 5 minutes, a solution of 5.63 g (22.2 mmol) iodine in 11 ml of DMF was added dropwise. After the addition was complete, stirring was continued for 15 minutes .
Subsequently the reaction mixture was poured into a solution containing 2.22 g of NaHSO₃, 22 ml of 25% NH₄OH and 333 ml of water. Crystallization started, filtration yielded 5.87 g of the unstable 3-indolyl-iodide, which was directly used in step ii.

### Scheme 17, step ii:

The 3-indolyl-iodide was dissolved in 33 ml of toluene, and in the following order were added: 33 ml of water, 22 ml 50% of NaOH and 0.71 g (2.22 mmol) of TBAB. While vigorously stirring, a solution of 2.8 g (24.4 mmol) of mesylchloride in 33 ml of toluene, was added. After the addition was complete, stirring was continued for 90 minutes . The reaction mixture was washed with water (2x), and the organic fraction was concentrated in vacuo, yielding 6.67 g of a light brown oil. This residu was purified by flash column chromatography (SiO₂, eluent: DCM/petroleum ether 2/3), yielding 3.92 g (almost white) of the corresponding pure N -mesyl-derivative.

### Scheme 17, step iii:

0.65 g (2 mmol) of the N-mesyl-derivative (from step ii), 0.13 g (2.4 mmol) of propargylalcohol, 55 mg (0.078 mmol) of (PPh₃)₂PdCl₂, 27 mg (0.141 mmol) of Cul, were taken up in 10 ml of triethylamine (degassed for 30 minutes ). This mixture was stirred for 5 hours under an nitrogen atmosphere. Subsequently, water and diethylether were added, the water fraction was extracted with diethylether. The combined organic fractions were washed with brine, and filtered over a water repellant filter, the dry filtrate concentrated in vacuo. The residu was purified by flash column chromatography (SiO₂, eluent: DCM/MeOH 97/3), yielding 0.40 g (76%) of the pure N-Ms-**Q17**-OH.

### Scheme 17, step iv:

0.40 g (1.52 mmol) of **Q17**-OH (from step iii), 480 mg (1.82 mmol) of PPh₃ and 600 mg (1.82 mmol) of tetrabromomethane, were taken up in 10 ml of DCM. The reaction mixture was stirred for 28 hours after which the reaction mixture was concentrated in vacuo and the residu purified by flash column chromatogr aphy (SiO₂, eluent: ethylacetate/petroleum ether 1/4), yielding 430 mg (86%) of a light yellow oil (solidifies on standing) containing N -Ms-**Q17**-Br.

### Synthesis of Q18, 51-52, 94-95:

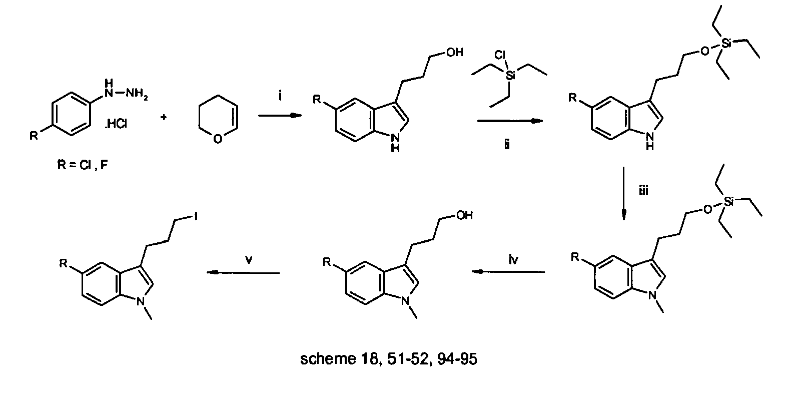

All starting hydrazines were commercially available.

### Scheme 18, 51-52, 94-95, step i:

### R=Cl

A stirred suspension of 4-Chlorophenylhydrazine monohydrochloride (25 g, 139 mmol) in 260 ml of 1,2-propanediol was heated on an oil bath of 110 °C. 3,4-dihydropyrane (12.5 ml, 136 mmol) was added dropwise for 15 minutes. The reaction mixture was stirred for 4.5 hours at 95-100 °C. After cooling to room temperature, 150 ml of 25% NaOH were added and stirring was continued for 10 minutes . 250 ml of MTBE were added and after additional stirring for 10 min., the MTBE-layer was separated and the aqueous layer was extracted 2x with MTBE. The combined organic layers were washed with H₂O, 5% NaHCO₃ and brine respectively. The organic layer was dried (Na₂SO₄). The drying agent was removed by filtration and the solvent by evaporation under reduced pressure. The residue was chromatographed (SiO₂) using EtOAc/petroleum ether 4/1 as the eluent to give 25.6 g (87%) of the indole as a brown oil, containing **Q95**-OH.

### Scheme 18, 51-52, 94-95, step ii:

To a stirred solution of the indole **Q95**-OH of step i (25.9 g, 123 mmol) and imidazole (8.71 g, 128 mmol) in 150 ml DMF at 0 °C, was added triethylsilylchloride (21.5 ml, 128 mmol). The reaction mixture was stirred for 3 hours at room temperature, after which H₂O and Et₂O were added. The layers were separated and the aqueous layer was extracted once with Et₂O. The combined Et₂O layers were washed with H₂O (3x) and brine respectively. The Et₂O fraction was dried (Na₂SO₄) and concentrated in vacuo to give 36.04 g (90%) of the silylated alcohol as a brown oil.

### Scheme 18, 51-52, 94-95, step iii:

To a stirred suspension of NaH (60%) (5.12 g, 128 mmol) in 100 ml of dry DMF, a solution of the silylated alcohol of step ii (36.04 g, 107 mmol) in 50 ml dry DMF was added dropwise. Stirring was continued at room temperature for 1 h. The reaction mixture was cooled to 0 °C and a solution of Mel (8.65 ml, 139 mmol) in 50 ml dry DMF was slowly added dropwise. After the addition was completed the reaction mixture was stirred at room temperature for 18 h. H₂O was added and the aqueous layer was extracted 3x with Et₂O. The combined Et₂O layers were washed with H₂O (3x) and brine (1x) respectively . The Et₂O fraction was dried (Na₂SO₄) and concentrated in vacuo. The residue was chromatographed (SiO₂) using CH₂Cl₂/PA 1/1 as the eluent to give 31.51 g (87%) of the methylated indole as a viscous liquid.

### Scheme 18, 51-52, 94-95, step iv:

A mixture of the methylated indole (31.5 g, 90 mmol) and 117 ml of 1.0 M (in THF) TBAF (117 mmol) was stirred at room temperature for 20 hours, after which H₂O and Et₂O were added. The Et₂O layer was separated and the aqueous layer was extracted once with Et₂O. The combined Et₂O layers were washed with H₂O (3x) and brine respectively. The Et₂O fraction was dried (Na₂SO₄) and concentrated in vacuo. 200 ml of petroleum ether were added to the residue and the resulting suspension was filtered by suction to give 17.19 g (85%) as an off white solid, containing **Q51**-OH.

### Scheme 18, 51-52, 94-95, step v:

The conversion of the resulting alcohols to the corresponding iodo derivatives was performed analogously to the procedure described in scheme 79-84 step iii.

**Q18**-OH, **Q52**-OH and **Q94**-OH can be synthesized analogously to the previous procedures.

### Synthesis of Q19-26:

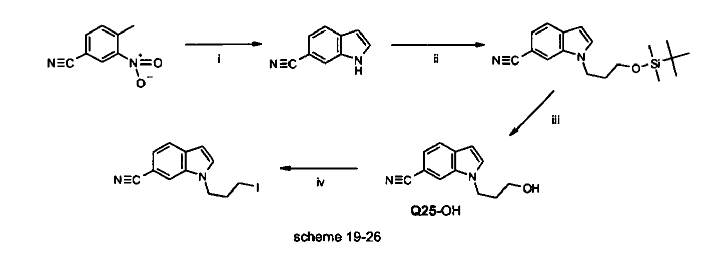

All starting materials were commercially available.

### Scheme 19-26 step i:

To a stirring solution of 3-nitro-p-tolunitrile (16.58 g, 102.3 mmol) in 55 ml DMF was added DMF-dimethylacetale (15.24 g, 128.1 mmol). The reaction mixture turned dark red and was stirred at 110 °C for 3 h. The solvent was removed under reduced pressure and taken up in a mixture of 300 ml EtOH and 300 ml acetic acid. The reaction mixture was heated to 60 °C and iron powder (33 g, 594 mmol) was added in portions. The reaction mixture was refluxed for 2 hours and filtered over a pad of Hyflo. Et₂O was added to the filtrate and the acidic layer was extracted with Et₂O (1x). The Et₂O fraction was concentrated in vacuo. The residue was chromatographed (SiO₂) with CH₂Cl₂ as the eluent to give 7.02 g (48%) of a solid, containing the 6-cyano-indole.

### Scheme 19-26 step ii:

To a stirring suspension of NaOH (60%) (1.13 g, 25.96 mmol) in 60 ml DMF under a nitrogen atmosphere was added 6-cyanoindole of step i (3.51 g, 24.72 mmol) in portions. After stirring at room temperature for 1 hour the 1 -(dimethyl-tert.butylsilyl)-3-bromo propane (6.30 ml, 27.29 mmol) was added dropwise at -5 °C. The reaction mixture is stirred at room temperature for 20 h. 400 ml H₂O and 400 ml Et₂O were added. The Et₂O layer was separated and the aqueous layer was extracted 1x with Et₂O. The combined Et₂O layers were concentrated in vacuo. The residue was chromatographed (SiO₂) with CH₂Cl₂/petroleum ether 3/1 as the eluent to give 5.50 g (71%) as a light yellow oil.

### Scheme 19-26 step iii:

Was performed analogously to step iv in scheme 79-84-b, and yielded **Q25**-OH.

### Scheme 19-26 step iv:

The conversion of the resulting alcohols to the corresponding iodo derivatives was performed analogously to the procedure described in scheme 79-84 step iii.

The 6-cyano-indole derivative **Q26**-OH was prepared according to the procedure described above. The indole, 6-Fluoroindole and 6-Chloroindole were commercially available and were further converted to the indole derivatives **Q19-24-**OH according to the procedures given above.

### Synthesis of Q27:

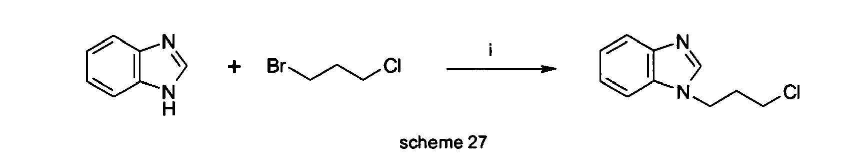

### Scheme 27 step i:

To a stirred suspension of NaH (55%) (0.48 g, 20 mmol) in 20 ml NMP at room temperature was added dropwise a solution of benzimidazole (1.18 g, 10 mmol) in 20 ml NMP. The reaction mixture turned light red and hydrogen forming was observed. After stirring at room temperature for 30 minutes 3 -chlorobromopropane (1.08 ml, 11 mmol) in 10 ml NMP was added dropwise. The reaction mixture was stirred at room temperature for 2 hours after which the reaction mixture was heated at 100 °C for 2 h. After additional stirring at room temperature for 72 h, H₂O and EtOAc were added. The layers were separated and the aqueous layer was extracted with EtOAc (2x). The combined organic layers were washed with brine (1x) and dried (MgSO₄). The drying agent was removed by filtration and the solvent by evaporation under reduced pressure to give 2.9 g of **Q27-**Cl (150%, still NMP present) as an oil. This was used in coupling reactions with amines.

### Synthesis of Q28-29:

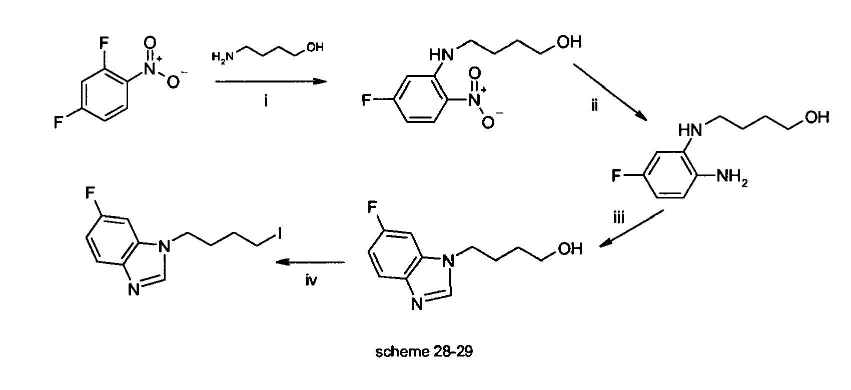

All reagents were commercially available.

### Scheme 28-29 step i:

To a stirring solution of 2,4-difluoronitrobenzene (8 g, 50.3 mmol) in 100 ml CH₃CN was added 4-aminobutanol (5.61 ml, 60.4 mmol) and DIPEA (20.9 ml, 120.7 mmol). The reaction mixture was stirred at room temperature for 72 h. The solvent was evaporated under reduced pressure and CH₂Cl₂ was added to the residue. The CH₂Cl₂ fraction was washed with H₂O (2x), dried (by a Water Repelling Filter) and the filtrate evaporated under reduced pressure. The residue was chromatographed (SiO₂) with Et₂O as the eluent to give 9.68 g (84%) of the amino -alkylated product.

### Scheme 28-29 step ii:

To a solution of the amino-alkylated product (from step i) (9.68 g, 42.5 mmol) in 250 ml EtOH (96%) was added 1 g 10% Pd/C after which the mixture was hydrogenated at room temperature (1 atm) for 3 h. The reaction mixture was filtered through a pad of Hyflo and the black filtrate was concentrated in vacuo under reduced pressure to give 8.42 g (100%) of the corresponding aniline.

### Scheme 28-29 step iii:

A mixture of the aniline (from step ii) (8.42 g, 42.5 mmol) in 25 ml formic acid (96%) was refluxed for 2.5 hours after which it was allowed to cool to room temperature. H₂O was added and after cooling, to the reaction mixture 50 ml of 50% NaOH was added. After stirring for 2 hours the aqueous layer was extracted with CH₂Cl₂. The CH₂Cl₂ fraction was dried (by a Water Repelling Filter) and concentrated in vacuo under reduced pressure. The residue was chromatographed (SiO₂) with CH₂Cl₂/MeOH 9:1 as the eluent to give 8.1 g (92%) of the benzimidazole.

### Scheme 28-29 step iv:

The conversion of the resulting alcohols to the corresponding iodo derivatives was performed according to the procedure described in scheme 79-84 step iii. In this case triphenylphosphine on solid support was used.

**Q28-**OH was prepared via the same procedure as described above.

### Synthesis of Q30:

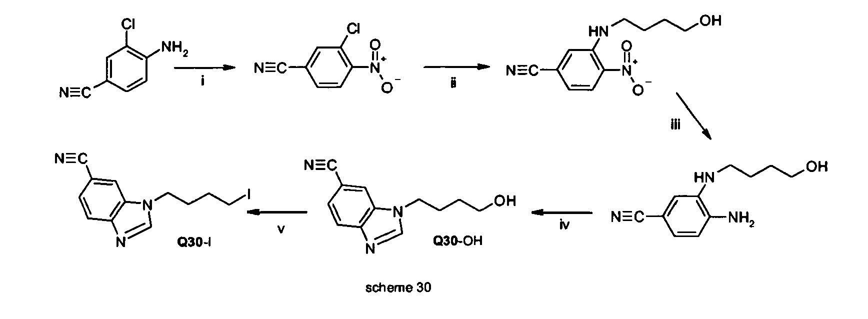

All reagents were commercially availab le.

### Scheme 30 step i:

A suspension of sodium borate tetrahydrate (32.5 g, 211.2 mmol) in 195 m! of acetic acid was heated until the temperature of the reaction mixture was above 50°C. The reaction temperature was kept this way while 2 -chloro-4-cyanoaniline (5.93 g, 38.9 mmol) was added in portions over 1 h. Stirring and heating were continued for 2 hours on an oil bath of 62°C. After cooling to room temperature the reaction mixture was poured into 1 L icewater. The aqueous layer was extracted with Et₂O (3x). The combined organic layers were washed with H₂O (2x) and dried (MgSO₄). The drying agent was removed by filtration and the solvent by evaporation under reduced pressure. The residue was chromatographed (SiO₂) with Et₂O/petroleum ether 1/3 as eluent to give 5.27 g (74%) of the oxidized product.

### Scheme 30 step ii:

To a stirring solution of 2-chloro-4-cyanonitrobenzene from step i (2.48 g, 13.6 mmol) in 12 ml DMF was cooled in ice. 4 -aminobutanol (5.50 ml, 59.3 mmol) was added and the reaction mixture was slowly allowed to warm to room temperature after which stirring was continued at room temperature for 72 h. H₂O was added and the aqueous layer was extracted with CH₂Cl₂ (2x) The combined organic layers were washed with H₂O (3x), dried (by a Water Repelling Filter) and evaporated under reduced pressure. The residue was chromatographed with Et₂O/petroleum ether 4:1 as eluent to give 2.6 g (49%) of the amino-alkylated product.

### Scheme 30 step iii:

Prepared according to step ii, in scheme 28-29.

### Scheme 30 step iv:

Prepared according step iii, in scheme 28-29.

### Scheme 30 step v:

Prepared according step iv, in scheme 28-29.

### Synthesis of Q31-34:

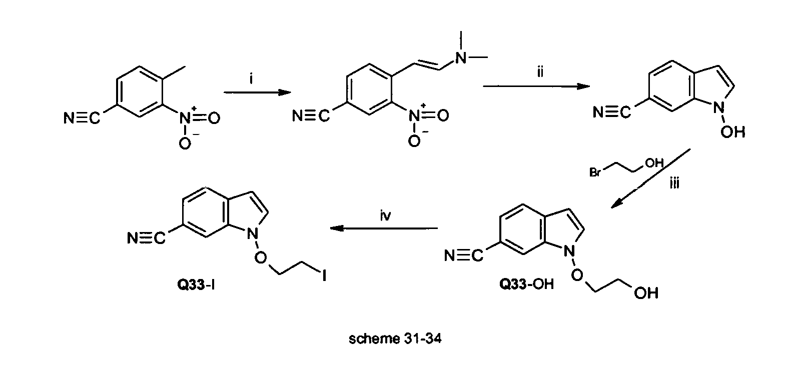

All reagents were commercially available.

### Scheme 31-34 step i:

To a stirring solution of 3-nitro-p-tolunitrile (8.1 g, 50 mmol) in 30 ml DMF was added DMF-dimethylacetale (13.3 ml, 100 mmol) and the reaction mixture was stirred at 120 °C for 3 h. The solvent was evaporated under reduced pressure and the residue was taken up in CH₂Cl₂. The CH₂Cl₂ fraction was washed with H₂O (2x), dried (by a Water Repelling Filter). The drying agent was removed by filtration and the solvent by evaporation under reduced pressure to give 10.6 g (98%) of the adduct.

### Scheme 31-34 step ii:

To a stirring emulsion of the adduct (from step i) (6 g, 27.6 mmol) in 175 ml Et₂O was added 8.1 g NH₄Cl and 29 g zinc granules (40 mesh). After stirring at room temperature for 2 hours 100 ml THF was added to dissolve the starting material. After an additional stirring for 6 hours the reaction mixture was filtered over a pad of Hyflo. Half of the resulting filtrate was used in the next step.

### Scheme 31-34 step iii:

To the filtrate of the former step ii was added 2-bromoethanol (7.9 ml, 112 mmol), Aliquat (0.6 g, 10 mol%) and 90 ml 10% NaOH. The reaction mixture was stirred at room temperature for 20 h. After separation of the layers, the aqueous layer was extracted with Et₂O (1x). The combined organic layers were washed with H₂O (4x) and dried (MgSO₄). The drying agent was removed by filtration and the solvent by evaporation under reduced pressure (by means of an oil pump). The residue was chromatographed (SiO₂, eluent: CH₂Cl₂ → CH₂Cl₂/Et₂O 4:1) to give 1 g (36%) of the corresponding alcohol **Q33-**OH.

### Scheme 31-34 step iv:

The conversion of the resulting alcohols to the corresponding iodo derivatives was performed according to the procedure described in scheme 79 -84 step iii.

**Q31-**OH, **Q32-**OH and **Q34**-OH were prepared analogously to the procedure described above.

### Synthesis of Q35:

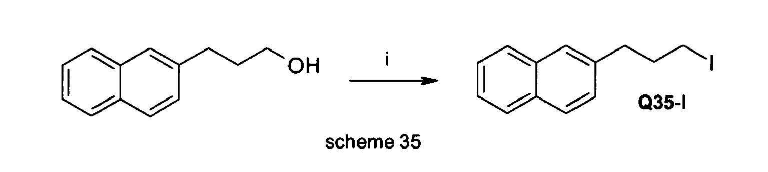

The naphtylpropylalcohol was prepared according to: Searles, *J.Amer.Chem.Soc.,* **73**(1951) 124.

### Scheme 35 step i:

The conversion of the resulting alcohol to the corresponding iodo derivative was performed according to the procedure described in scheme 79 -84 step iii.

### Synthesis of Q36:

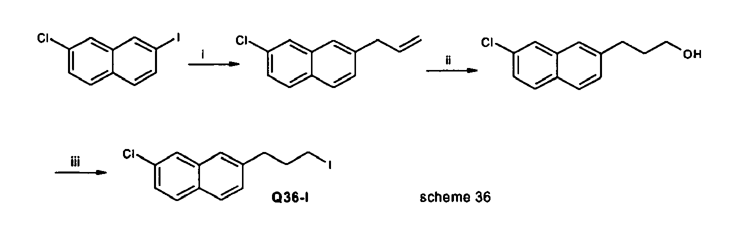

2-chloro-7-iodo-naphtalene was prepared according to the literature (Beattie; Whitmore; J. Chem. Soc. 1934, 50,51,52)

### Scheme 36 step i:

A 100 ml roundbottom flask under an nitrogen atmosphere was charged with 2-chloro-7-iodo-napthalene (11 mmol, 3,60g), allyl-tributyltin (13 mmol, 4.30g, 3.96 ml), tetrakis(triphenylphosphine)palladium(0) (0.55 mmol, 0.635g) and 10 ml degassed benzene. The mixture was heated to relfux under a nitrogen atmosphere and after 20 hours another portion of tetrakis(triphenylphosphine)palladium(0) (0.55 mmol, 0.635g) was added. The mixture was again heated at reflux for 20 hours after which it was allowed to cool to room temperature after which it was poured into 70 ml of a 10% KF-solution. After 30 min stirring at room temperature the suspension was filtered over Hyflo Supercel^{®}. The filtrate was washed with water, brine and dried (Na₂SO₄). Column chromatography on silica gel (eluens 1/9 toluene/petroleum ether) afforded almost pure 2-allyl-7-chloro-napthalene (1.80g, 80%).

### Scheme 36 step ii:

A 100 ml threeneck roundbottom flask under a nitrogen atmosphere was charged with 2-allyl-7-chloro-napthalene (1.80g, 8.9 mmol) and 12 ml of dry THF. The mixture was cooled in an ice-bath and borane-THF (3.05 mmol, 3.05 ml 1.0 M borane in THF) was added dropwise in about 20 minutes After the addition the mixture was allowed to warm to room temperature and stirred for 20 hours. 3.0 N NaOH solution (2.65 mmol, 0.89 ml) was then added to the solution and the mixture was cooled in a waterbath while adding 30% hydrogenperoxide (10.62 mmol, 1.1 ml) dropwise at such a rate that the temperature did not exceed 30 °C. After the addition the mixture was stirred for 6 hours at room temperature.

Water and diethyl ether were added and the organic layer was separated. The water layer was extracted again with ethyl ether and the combined organic extracts were washed with water, brine and dried (Na₂SO₄). The drying agent was removed by filtration and the solvent by evaporation *in vacuo.* Flash column chromatography on silica gel (eluent: 1/99 methanol/dichloromethane) afforded 3-(7-chloro-napthalene-2-yl)-propan-1-ol (0.79 g, 40%) **Q36-**OH.

### Scheme 36 step iii:

The conversion of the resulting alcohol to the corresponding iodo derivative was performed according to the procedure described in scheme 79-84 step iii, yielding **Q36-**I.

### Synthesis of Q37:

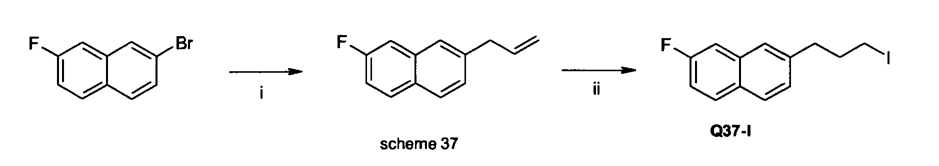

The fluorobromonaphtalene was prepared according to: Adcock,W. et al., Aust.J.Chem., 23(1970)1921-1937.

### Scheme 37 step i:

To a stirred suspension of magnesium turnings (0.49 g, 20 mmol) and 0.1 ml 1,2 - dibromoethane in 20 ml THF was added the fluoronaphtalene (0.45 g, 2 mmol) in one time. After the start of the grign ard a solution of the fluoronaphtalene (4.06 g, 18 mmol) in 25 ml THF was slowly added dropwise. The temperature rose during the addition to 40 °C. The reaction mixture was stirred at room temperature for 2 hours until all the magnesium had disappeared. A freshly prepared solution from LiCl and CuCN in THF was added dropwise at -10 °C which resulted in a dark green solution. At the same temperature was added dropwise a solution of allyl bromide (1.9 ml, 22 mmol) in 15 ml THF. After the complete addition the reaction mixture was stirred at -10-0 °C for 30 minutes. The green color disappeared and stirring was continued at room temperature for 20 h. The reaction mixture was poured into 200 ml of saturated NH₄Cl and extracted with CH₂Cl₂ (3x). The combined organic layers were washed with brine and dried (MgSO₄). The drying agent was removed by filtration and the solvent by evaporation under reduced pressure. The residue was chromatographed (SiO₂) using petroleum ether as eluent to give 1.65 g (44%) of the corresponding allyl-fluoronaphtalene.

### Scheme 37 step ii:

To a cooled stirring solution of the allyl-fluoronaphtalene (1.65 g, 8.8 mmol) in 10 ml THF at -5 °C was slowly added dropwise 3.05 ml 1.0 M Borane.THF-complex. After stirring for 20 minutes at the same temperature and additional stirring at room temperature iodine (2.11 g, 8.6 mmol) was added in one time. 3.1 ml of a freshly prepared 2.7 M solution of sodium metal in MeOH) was slowly added dropwise (exothermic) after which the reaction mixture is stirred at room temperature for 20 h. 75 ml NaHSO₃ was added and the aqueous layer was extracted with CH₂Cl₂ (3x). The organic layer was washed with brine (1x) and dried (MgSO₄). The drying agent was removed by filtration and the solvent by evaporation under reduced pressure. The residue was chromatographed (SiO₂) using petroleum ether as eluent to give 1.25 g (46%) of the iodide **Q37**-I as a white solid.

### Synthesis of Q38-43, Q47-48:

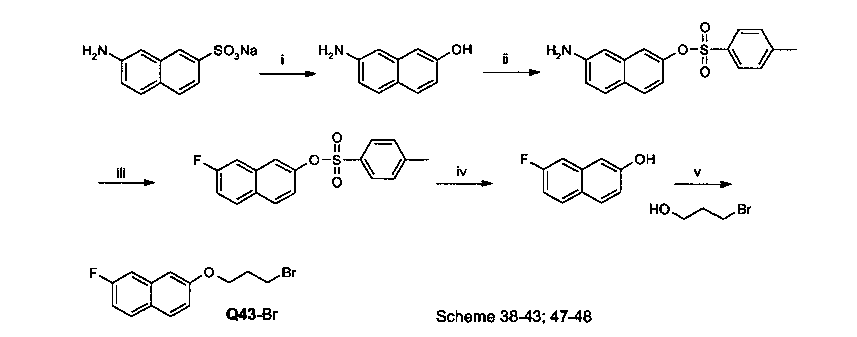

### Scheme 38-43, 47-48 step i:

A mixture of KOH pellets (140 g, 2.5 mol) and 10 ml H₂O in a nickel crucible was heated to 250 °C with a Bunsen burner while being stirred with a stainless steel stirrer. The flame is removed and 7-amino-2-naphtalenesulfonic acid sodium salt (0.245 mol, 60.0 g) was added to the clear liquid in 3 portions. The clear liquid changes into a thick black slurry which is again strongly heated with a Bunsen burner. At about 280°C gas evolved and the temperature of the mixture quickly rises to 310-320°C. This temperature was maintained for 8 minutes after which the mixture was allowed to cool to about 200 °C. The thick black paste was carefully transferred to a 3 litre beaker filled with ice.

The product of 2 runs were combined and neutralized with concentrated HCl under cooling with an ice-salt bath. The suspension wa filtered and the black solid wa washed with 4 500 ml portions of 1.0 *N* HCl and discarded. The brown, clear filtrate that is obtained was cooled in an ice-salt bath and KOH-pellets are added until a light suspension was obtained. After addition of a saturated NH₄OAc-solution the green-grey solid fully precipitates and was collected through filtration to obtain 7-amino-naphtalene-2-ol (27.9 g, 36%) after drying in the air.

### Scheme 38-43, 47-48 step ii:

7-amino-naphtalene-2-ol (0.169 mol, 27.0 g) is suspended in 750 ml DCM and TEA (0.169 mol, 17.2 g, 23.6 ml) was added. The mixturewais stirred for 30 min at room temperature after which it was cooled to -5°C in an ice-salt bath. A solution of p-Tosylchloride (0.17 mol, 32.4 g) in 250 ml DCM was added over a period of 2.5 hours at -5-0 °C. The mixture was stirred for 10 minutes at -5-0 °C after which it was allowed to warm to room temperature and stirred for 18 hours.
1 L of H₂O was added to the mixture and the resulting suspension was filtered over Hyflo Super Cel^{®} and the filtrate was transferred to a separatory funnel. After extracting the organic layer, the water-layer was again extracted with DCM (2x). The combined organic layers are washed with brine, dried (Na₂SO₄) and concentrated *in vacuo* to give 51.5 g of a black oil which was purified by column chromatography on silica gel (eluens 1/1 ethylacetate/petroleum ether) to afford toluene-4-sulfonic acid-7-amino-napthalene-2-yl-ester (12.1 g, 23%).

### Scheme 38-43, 47-48 step iii:

A 500 ml threeneck roundbottom flask made from PFA was charged with 100 g Pyridine/HF complex (30:70 %w/w) and cooled to -10 °C with an ice/EtOH bath. toluene-4-sulfonic acid-7-amino-napthalene-2-yl-ester (38.6 mmol, 12.1g) was added in one portion and the mixture was stirred for 10 minutes after which a clear purple solution was obtained. This solution was cooled to < -30 °C in an dry-ice cooling bath and sodium nitrite (42.5 mmol, 2.93 g, dried by heating at 140 °C for 3 days) was added in one portion. The dry-ice bath was replaced by a normal ice-bath and the mixture was stirred at 0°C for 20 minutes after which it was heated to 55-60°C on an oilbath (evolution of nitrogen was observed). After 1.5 hours nitrogen evolution ceased and the mixture was allowed to cool to room temperature and poured into a large beaker filled with ice. The mixture was transferred to a separatory funnel and extracted 3 times with DCM. The organic layers where pooled together, washed with brine and dried (Na₂SO₄). Concentration *in vacuo* afforded 10.4 g of a red oil which was purified by flash column chromatography on silica gel (eluens 1/4 ethylacetate/petroleum ether) to give toluene-4-sulfonic acid-7-fluoro-napthalene-2-yl-ester (7.1 g, 58%)

### Scheme 38-43, 47-48 step iv:

A 500 ml roundbottom flask protected with a CaCl₂-tube was charged with toluene-4-sulfonic acid-7-fluoro-napthalene-2-yl-ester (22.4 mmol, 7.1 g) and 200 MeOH. The suspension was heated until a clear solution was obtained and then cooled down to room temperature in a waterbad to afford a fine suspension. Magnesium (179 mmol, 4.36 g) was added to the mixture which was then stirred for 4 hours at room temperature. The brown suspension was cooled in an ice-EtOH bath and acidified with 6N HCl and then concentrated *in vacuo.* The mixture was transferred to a separatory funnel and extracted 3 times with ethylether. The organic extracts are pooled together, washed with brine and dried (Na₂SO₄). The drying agent was removed by filtration and the solvent by evaporation *in vacuo*. Flash column chromatography on silica gel (eluens dichloromethaan) afforded unpure 7 -fluoro-napthalene-2-ol (4.69 g) as an off white solid. This solid was dissolved in DCM and extracting 3 times with 2N NaOH-solution. The basic extracts were combined and acidified with 3N HCl while cooling with an ice bath. White crystals precipitated from the solution and were collected by filtration and dried in the air to afford pure 7 - fluoro-napthalene-2-ol (3.16 g, 87%)

### Scheme 38-43, 47-48 step v:

This Mitsunobu step was done analogously to step vi in scheme XIII, yielding **Q43-**Br.

**Q38** was synthesized as **Q38**-I**, Q39-42** and **Q47-48** derivatives were prepared similarly to the above described procedures (as bromides).

### Synthesis of Q46, Q49:

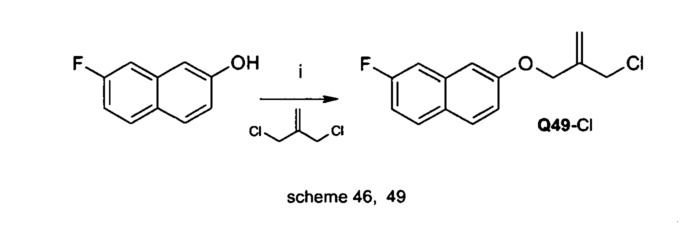

### Scheme 46 49 step i:

A mixture of 7-fluoro-2-naphtol (see Scheme 38-43, 47-48 step iv) (0.62 g, 3.82 mmol), the alkene (1.11 ml, 9.56 mmol) and K₂CO₃ (1.58 g, 11.5 mmol) in 35 ml CH₃CN was refluxed for 3 hours after which was was cooled to room temperature and evaporated under reduced pressure. The residue was taken up in H₂O and Et₂O and extracted with Et₂O (2x). The combined organic layers were washed with H₂O (1x) and brine (1x) after which it was dried (Na₂SO₄). The drying agent was removed by filtration and the solvent by evaporation under reduced pressure. The residue was chromatographed (SiO₂) with CH₂Cl₂/petroleum ether 1/5 as eluent to give 0.56 g (58%) of the fluoronaphthol derivative **Q49-**Cl as a colorless oil.

### Synthesis of Q50:

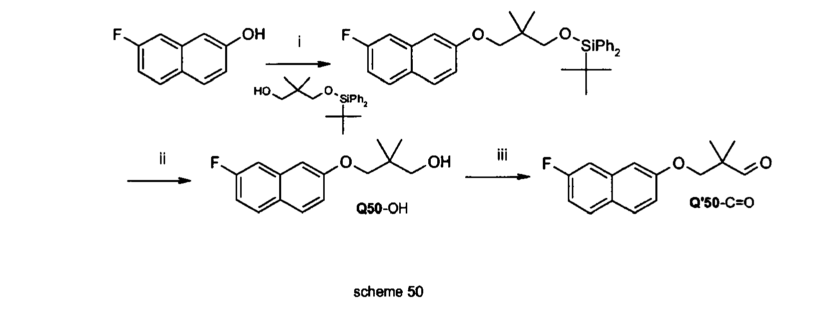

### Scheme 50 step i:

For the fluornaphtol, see Scheme 38-43, 47-48 step iv. This Mitsunobu reaction was performed analogously to step vi in scheme XIII.

### Scheme 50 step ii:

This step can be performed similar to step iv in scheme 79 -84 b, and yielded **Q50-**OH.

### Scheme 50 step iii:

**Q50**-OH was oxidized following the procedure of step i in scheme B2. The product, **Q'50**-C=O was used in the reductive alkylation of amines.

### Synthesis of Q53-58:

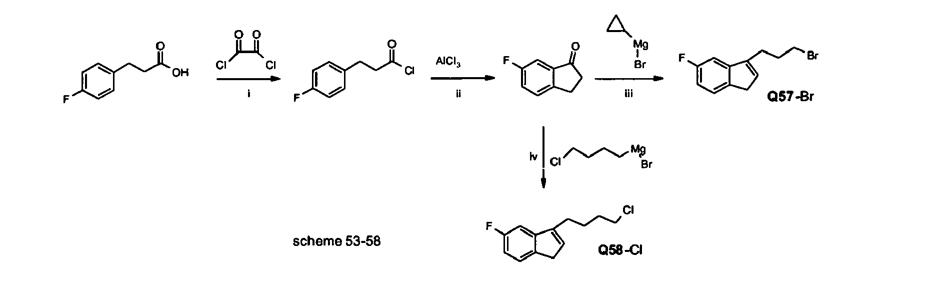

The starting acid and reagents were commercially available. The Cl -C4-MgBr was prepared according to: C.R. Hebd, Seances Acad. Ser. C, 268 (1969)1152-1154.

### Scheme 53-58 step i:

To a solution of the acid (25 g, 148.8 mmol) in 140 ml benzene was added 0.07 ml DMF after which oxalylchloride was added all at once. Immediate foaming of the reaction mixture was observed. The reaction mixture was stirred for at room temperature 18 hours and the solvent was removed by evaporation under reduced pressure. Acetonitrile was added to the residue for co-evaporation and again removed by evaporation under reduced pressure to give 27.75 g (100%).

### Scheme 53-58 step ii:

AlCl₃ (27.8 g, 208 mmol) was suspended in 200 ml 1,2-dichloroethane. The mixture was cooled under a nitrogen atmosphere to 0-5 °C and a solution of the acid chloride (27.75 g, 148.8 mmol) in 140 ml 1,2-dichloroethane was added dropwise in 1 h. The cooling bath was removed and after stirring for 30 min., stirring was continued for 2 hours at 70 °C. After cooling to room temperature the reaction mixture was poured into a mixture of ice and 330 ml concentrated HCl (36 -38%).

The aqueous layer was extracted with CH₂Cl₂ and the resulting organic layer was washed with H₂O (2x), 5% NaHCO₃ and brine. The organic layer was dried (MgSO₄). The drying agent was removed by filtration and the solvent by evaporation under reduced pressure to give 19.02 g (85%).

### Scheme 53-58 step iii:

To a cooled solution of 0.5M cyclopropyl magnesiumbromide in THF (100 ml, 50 mmol) at 15°C was added a solution of the ketone (5.3 g, 35.3 mmol) in 40 ml THF. The reaction mixture was stirred at reflux for 2 hours after which was was cooled in an ice bath. 50 ml saturated NH₄Cl was added dropwise and the aqueous layer was extracted with Et₂O. The Et₂O was washed with brine (1x), dried (MgSO₄) and evaporated under reduced pressure. The residue was dissolved in 85 ml acetic acid and 62 ml of a 20% HBr solution was added. The reaction mixture was stirred for 20 h. H₂O was added and the aqueous layer was extracted with CH₂Cl₂. The organic layer was further washed with H₂O (1x) and 5% NaHCO₃ (1x). The organic layer was dried (by a Water Repelling Filter) and evaporated under reduced pressure. The residue was chromatographed with CH₂Cl₂/petroleum ether 2.5/97.5 as eluent to give 4.44 g (49%) of the indene **Q57-**Br.

### Scheme 53-58 step iv:

Was prepared according to the procedure as described for step iii, yielding **Q58**-Cl

**Q53, Q54, Q55,** and **Q56** derivatives were made analogously to the above described procedure.

### Synthesis of Q59:

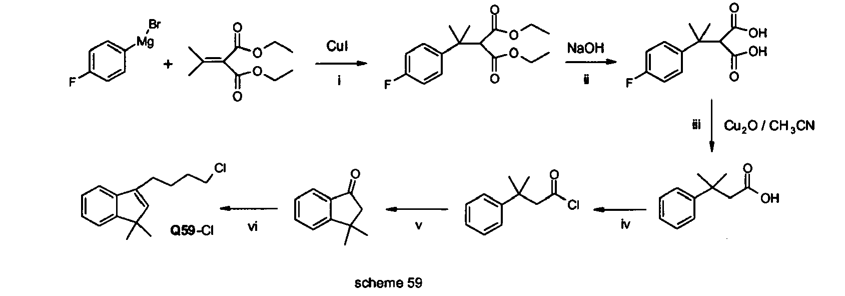

The starting materials were commercially available.

### Scheme 59 step i:

A mixture of the Grignard reagent (90 ml, 90 mmol) and Cul (18 mg, 0.02 mmol) was stirred for 15 minutes after which it was cooled in an ice bath. A solution of the di-ester (18.9 ml, 96.7 mmol) in 25 ml THF was added in 90 min and the reaction mixture was stirred at 0 °C for 2 h. 100 ml saturated NH₄Cl was added dropwise and the aqueous layer was extracted with Et₂O. The Et₂O fraction was washed with brine (1x) and dried (MgSO₄). The drying agent was removed by filtration and the solvent by evaporation under reduced pressure. The residue was chromatographed with CH₂Cl₂/petroleum ether 1/1 as eluent to give 26.17 g (98%) of the adduct.

### Scheme 59 step ii:

To a stirring solution of the adduct (26.17 g, 88.4 mmol) in 222 ml EtOH was added 265 ml 10% NaOH. The reaction mixture was refluxed for 3 hours and the solvent was evaporated under reduced pressure. The residue was cooled in ice and acidified with concentrated HCl (36-38%). The aqueous layer was extracted with EtOAc. The EtOAc fraction was washed with brine (1x) and dried (MgSO₄). The drying agent was removed by filtration and the solvent by evaporation under reduced pressure to give 20.9 g (99%) of the di -acid.

### Scheme 59 step iii:

A mixture of the di-acid (20.9 g, 87.1 mmol) and Cu₂O (0.62 g, 4.34 mmol) in 600 ml CH₃CN was refluxed for 16 h. The solvent was removed by evaporation under reduced pressure and 125 ml 3N HCl was added to the residue. The aqueous layer was extracted with EtOAc. The EtOAc fraction was washed with brine (1x) and dried (MgSO₄). The drying agent was removed by filtration and the solvent by evaporation under reduced pressure to give 16.9 g (99%) of the de -carboxylated product.

### Scheme 59 step iv:

Was prepared according to step i in scheme 53 -58.

### Scheme 59 step v:

Was prepared according to step ii in scheme 53 -58.

### Scheme 59 step vi:

Was prepared according to step iii in scheme 53 -58, yielding **Q59-**Cl.

### Synthesis of Q60-61:

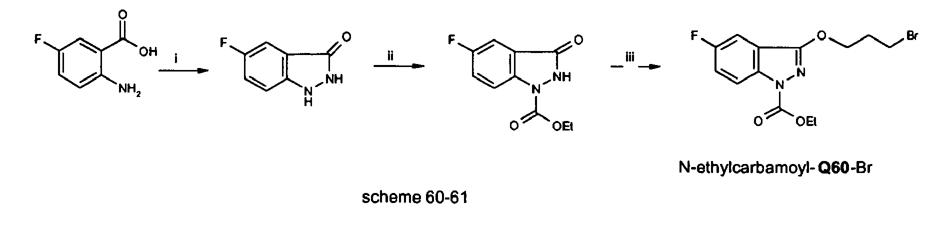

### Scheme 60-61 step i:

A 3 litre beaker was charged with 2-amino-5-fluoro-benzoic acid (64 mmol, 10 g), 100 ml H₂O and 110 ml concentrated HCl and the suspension was cooled to 0°C in an ice/aceton bath. A solution of natrium nitrite (64 mmol, 4.44 g) in 68 ml H₂O was added dropwise to the mixture while the temperature was maintained at below 3 °C. After the addition was complete the brown solution was added in portions over 20 minutes, under a stream of sulfurdioxide, to a solution of 760 ml H₂O saturated with sulfurdioxide cooled at 0-5°C with an ice-bath. After the addition was complete the ice-bath was removed and the solution was allowed to warm to room temperature while the stream of sulfurdioxide was maintained. After 1 hour the supply of sulfurdioxide was discontinued and the solution was allowed to stand at room temperature overnight. To the dark yellow solution which was obtained was added 620 ml concentrated HCl and after cooling the mixture a yellow precipitate separates which was collected on a cooled buchner funnel. The solid was suspended in a solution of 2 ml concentrated HCl and 200 ml H₂O and the mixture was heated to reflux. After a time the solid dissolves and a clear solution was obtained. After 1.5 hours of reflux a orange/brown sol id has crystallized and the mixture was allowed to cool to room temperature and was concentrated to about 50 ml *in vacuo.* The solid was collected and dried in the air to afford 5-fluoro-1,2-dihydro-indazol-3-one (5.05 g, 52%)

### Scheme 60-61 step ii:

5-fluoro-1,2-dihydro-indazol-3-one (32 mmol 5.05 g) was suspended in 30 ml pyridine and under cooling with an ice-bath chloroethylformiate (64 mmol, 6.94 g, 6.09 ml) was added dropwise. The mixture was heated to reflux for 3 hours and was then allowed to cool to room temperature ad concentrated *in vacuo* to afford a dark red oil which crystallizes after the addition of water. The solid was filtered and dried in the air to afford the corresponding urethane (5.52 g, 77%)

### Scheme 60-61 step iii:

To 20 ml toluene under a nitrogen atmosphere was added the urethane derivative (from step ii) (0.45 g, 2 mmol), 3-bromopropanol (0.18 ml, 2.1 mmol), Bu₃P (0.40 g, 2 mmol) and ADDP (0.5 g, 2 mmol). After the addition of ADDP the solution turned clear. The reaction mixture was heated at 85 °C for 20 hours and cooled to room temperature. 2N NaOH and EtOAc were added and the aqueous layer was extracted with EtOAc (2x). The combined organic layers were washed with 2N NaOH (1x), H₂O (1x) and brine (1x) after which the EtOAc was dried (Na₂SO₄) and evaporated under reduced pressure. The residue was chromatographed with CH₂Cl₂/MeOH 99:1 as eluent to give 0.22 g (32%) of the alkylated indazol -3-one. The latter compound can be coupled to an amine (see scheme A2, step i). The urethane moiety of the coupling product can be removed by using K₂CO₃ (3.5 eq) in a mixture of 26 ml of MeOH/DME/H₂O (5/1/1) per mmol of urethane derivative.

The **Q61** analogue can be synthesized as well, as described above.

### Synthesis of Q62-67:

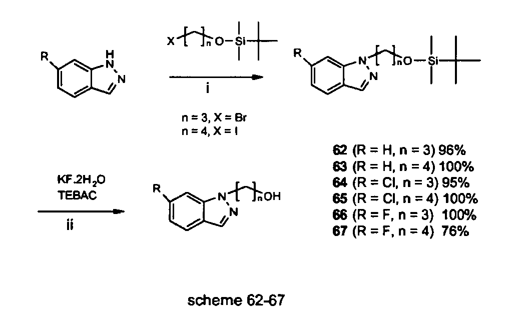

The indazoles were preapared according to Christoph Rüchardt, Volkert Hassemann; *Liebigs Ann. Chem*.; (1980) 908-927.

### Scheme 62-67, step i:

### 64; R=Cl, n=3:

NaH (55%) (2.14 g, 49.15 mmol) was suspended in 70 ml of dry DMF under a N ₂ atmosphere. 6-chloro-indazole (7,5 g, 49,15 mmol) was added at room temperature. The mixture was stirred for 1 hour before cooling with an ice bath and (3 -bromo-propoxy)-tert-butyl-dimethyl-silane (11.4 ml, 49.15 mmol) was added dropwise. After stirring for an additional 15 minutes the mixture was allowed to reach room temperature, stirring was continued for another 8 hours. Subsequently, the mixture was concentrated in vacuo and the residue was dissolved in DCM, the organic layer was then washed with water (3x). The organic layer was concentrated in vacuo. The crude product was purified by column chromatography on silica gel (SiO₂, eluent: petroleum ether/diethyl ether 5/1 ? 4/1) to afford the N1 substituted indazole in 61% yield.

### Scheme 62-67, step ii:

To a stirred solution of KF.2H₂O (4.3 g, 45,24 mmol) and benzyl tri-ethyl ammonium chloride (7.6 g, 33.18 mmol) in 300 ml acetonitrile, the N1 substituted indazole (from step i) (9.8 g, 30.16 mmol) was added. The mixture was warmed to reflux and stirred for 8 hours. The solvent was evaporated and DCM was added to the residue. The organic layer was washed with water (3x). De organic layer was concentrated in vacuo. The crude product was purified by flash chromatography on silica (eluent: diethylether → 1% MeOH in diethylether) to afford the 3-(indazol-1-yl)-propanol in 95% yield.

The other indazolyl alcohols were prepared analogously. In step ii, tetrabutyl ammonium chloride in THF can be used instead of the combination KF.2H₂O/ benzyl tri-ethyl ammonium chloride.

### Synthesis of Q68-78, Q85-86, Q91-93:

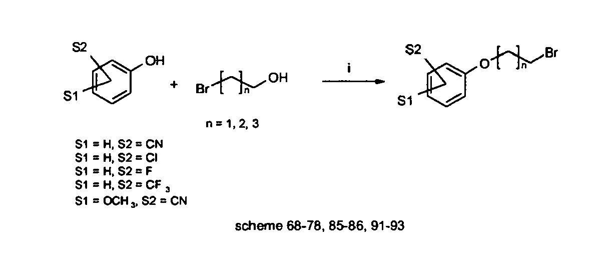

All starting phenols (except where S1= 2-OCH3, S2= 5-CN, see scheme 68-78, 85-86, 91-93; b) and alcohols were commercially available.

### Scheme 68-78, 85-86, 91-93; step i:

To a stirred solution of 3-fluorophenol (7 ml, 77.43 mmol), PPh₃ (26.4 g, 101 mmol) and 4-bromo-1-butanol (13.35 ml, 123.9 mmol) in 275 ml Toluene at 0 °C under an nitrogen atmosphere, was added dropwise a solution of DIAD (30.46 ml, 155 mmol) in 60 ml toluene (the temperature was kept between 0 °C and 5 °C during the addition). The reaction mixture was stirred for 20 hours at room temperature. The solution was evaporated under reduced pressure and 300 ml of Et₂O/petroleum ether 1:1 was added to the residue. The precipitate which formed during stirring for 30 minutes at room temperature was filtered off and washed 2 times with Et₂O/petroleum ether 1:3 (50 ml). The filtrate was evaporated under reduced pressure and the residue was chromatographed using DCM/petroleum ether 1:4 as eluent to give 18.39 g (96%) of the ether Q76-Br as a colorless oil.

### Synthesis of phenols with S1= 2-OCH₃ and S2= 5-CN:

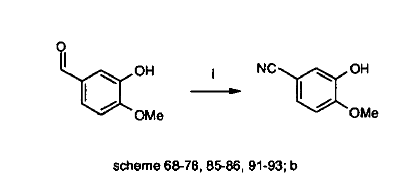

### Scheme 68-78, 85-86, 91-93; b, step i:

A mixture of 3-hydroxy-4-methoxybenzaldehyde (31.4 g, 206.6 mmol) and hydroxylamine monohydrochloride (18.7 g, 268.6 mmol) in 165 ml formic acid was refluxed for 1 h. The reaction mixture was cooled to room temperature and 1 litre of ice water was added. The precipitate was filtered and the residu washed with ice water. The solid was further dried by co-evaporation with acetonitrile yielding 13.84 g (45%) of a solid, containing the 2-methoxy-5-cyano-phenol.

### Synthesis of Q79-84:

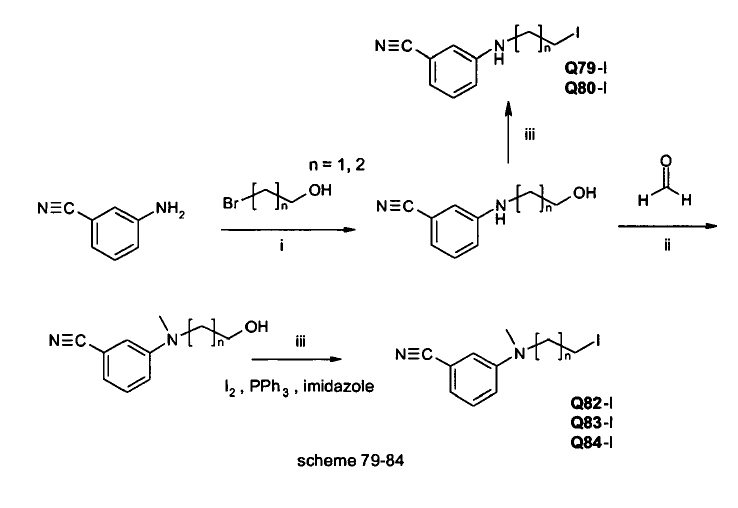

### Scheme 79-84 step i:

For n =2 and 3-cyanoaniline:
A mixture of 3-cyanoaniline (2.95 g, 25 mmol), Nal (7.5 g, 50 mmol), DIPEA (4.3 ml, 25 mmol) and 3-bromo-1-propanol (2.25 ml, 25 mmol) in 50 ml butyronitrile was refluxed for 24 h. The solvent was evaporated under reduced pressure. H₂O and CH₂Cl₂ were added to the residue and after separation the organic layer was dried (Na₂SO₄). The drying agent was removed by filtration and the solvent by evaporation under reduced pressure. The residue was chromatographed (SiO₂) using CH₂Cl₂/CH₃OH/NH₃ 92/7.5/0.5 as the eluent to give 3.45 g (78.4%) of the N-alkylated product **Q80-**OH as an oil.

### Scheme 79-84 step ii:

To a stirred solution of the N-alkylated product from step i (3.3 g, 18.8 mmol), 37% formaldehyde solution (14 ml, 188 mmol) and NaCNBH₃ (3.7 g, 56.4 mmol) in 70 ml CH₃CN was added dropwise 1.8 ml glacial acetic acid in 20 minutes . The reaction mixture was stirred for 2 hours at room temperature . Another 1.5 ml glacial acetic acid was added dropwise and stirring was continued for 30 minutes at room temperature . Et₂O was added to the reaction mixture and the ether fraction was washed 2 times with 1N NaOH. The combined organic fractions were dried (Na₂SO₄). The drying agent was removed by filtration and the solvent by evaporation under reduced pressure. The residue was chromatographed (SiO₂) using CH₂Cl₂/CH₃OH/NH₃ 92/7.5/0.5 as the eluent to give 1.80 g (50.4%) of the N-methylated alcohol **Q83-**OH as a thick oil.

### Scheme 79-84 step iii:

PPh₃ (3.1 g, 11.9 mmol) and imidazole (0.83 g, 11.9 mmol) were dissolved in 100 ml CH₂Cl₂. I₂ (3.1 g, 11.9 mmol) was added and the resulting suspension was stirred for 20 minutes at room temperature . A solution of the alcohol **Q83-**OH (from step ii)(1.8 g, 9.47 mmol) in 8 ml CH₂Cl₂ was added dropwise and the reaction mixture was stirred for 20 hours at room temperature. H₂O was added and after separation the CH₂Cl₂ fraction was dried (Na₂SO₄). The drying agent was removed by filtration and the solvent by evaporation under reduced pressure. The residue was chromatographed (SiO₂) using CH₂Cl₂ as eluent to give 2.18 g (76.8%) of the iodide **Q83**-I as a thick oil.

**Q81** needed another approach:

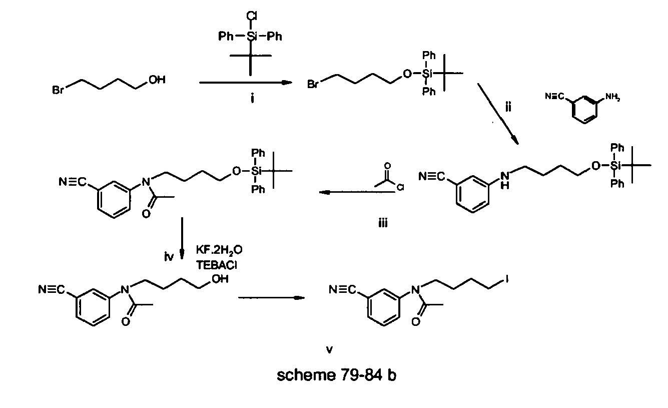

### Scheme 79-84 b step i:

To a stirred solution of 4-bromo-1-butanol (12 g, 78.4 mmol), imidazole (5.86 g, 86.2 mmol) in 70 ml DMF was added tert-butyldiphenylsilylchloride (20.4 ml, 78.4 mmol). The reaction mixture was stirred for 72 hours at room temperature The DMF was removed by evaporation under reduced pressure after which H₂O and CH₂Cl₂ were added. The CH₂Cl₂ fraction was dried (Na₂SO₄). The drying agent was removed by filtration and the solvent by evaporation under reduced pressure. The residue was chromatographed (SiO₂) using CH₂Cl₂/petroleum ether 1:4 as eluent to give 16.6 g (54.2%) of the silylated alcohol as an oil.

### Scheme 79-84 b step ii:

A mixture of 3-cyanoaniline (4.53 g, 38.36 mmol), silylated alcohol (from step i)(15 g, 38.36 mmol), DIPEA (6.64 ml, 38.36 mmol) and Nal (11.5 g, 76.73 mmol) in 400 ml butyronitrile was refluxed for 24 h. The solvent was evaporated under reduced pressure. H₂O and CH₂Cl₂ were added to the residue and after separation the organic layer was dried (Na₂SO₄). The drying agent was removed by filtration and the solvent by evaporation under reduced pressure. The residue was chromatographed (SiO₂) using CH₂Cl₂ as eluent to give 7.17 g (43.7%) of the alkylated aniline as an oil.

### Scheme 79-84 b step iii:

A mixture of the alkylated aniline (from step ii)(1 g, 2.34 mmol), acetyl chloride (0.17 ml, 2.34 mmol), DIPEA (0.40 ml, 2.34 mmol) in 15 ml CH₂Cl₂ was stirred at room temperature for 1 h. The solvent was evaporated under reduced pressure and the residue was chromatographed (SiO₂) using CH₂Cl₂/MeOH 98:2 as the eluent to give 0.88 g (80%) of the acylated aniline as a thick oil.

### Scheme 79-84 b step iv:

A mixture of the acylated aniline (from step iii)(0.88 g, 1.87 mmol), KF.2H₂O (0.26 g, 2.81 mmol), benzyltriethylammoniumchloride (0.37 g, 2.06 mmol) in 21 ml CH₃CN was refluxed for 4 h. The solvent was evaporated under reduced pressure. H₂O and CH₂Cl₂ were added to the residue and after separation the organic layer was dried (Na₂SO₄). The drying agent was removed by filtration and the solvent by evaporation under reduced pressure. The residue was chromatographed (SiO₂) using CH₂Cl₂/CH₃OH/NH₃ 92/7.5/0.5 as the eluent to give 0.31 g (71.4%) of the alcohol as a thick oil.

### Scheme 79-84 b step v:

The conversion of the resulting alcohols to the corresponding iodo derivatives was executed according to the procedure described in scheme 79 -84 step iii. The iodo derivatives can be coupled to amines (see Scheme A2, step i), the coupling product deacetylated by concentrated HCl in THF at reflux for 24 hours. The aniline NH moiety of the deacetylated product can be methylated by the use of methyliodide and K₂CO₃ in acetone.

### Synthesis of Q87-88:

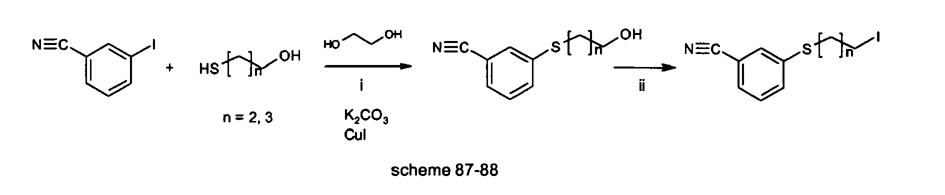

### Scheme 87-88 step i:

The starting iodobenzene and alcohols were commercially available.

A mixture of 3-iodobenzonitrile (2.29 g, 10 mmol), 4-mercapto-1-butanol (1.03 ml, 10 mmol), ethylene glycol (1.12 ml, 20 mmol), K₂CO₃ (2.78 g, 20 mmol) and Cul (95 mg, 0.5 mmol) in 40 ml 2-propanol under a nitrogen atmosphere was refluxed for 24 h. The solvent was evaporated under reduced pressure. Aqueous NH₄OH and CH₂Cl₂ were added to the residue and after separation the organic layer was dried (Na₂SO₄). The drying agent was removed by filtration and the solvent by evaporation under reduced pressure. The residue was chromatographed (SiO₂) using CH₂Cl₂/CH₃OH/NH₃ 96:3.75:0.25 as the eluent to give 1.40 g (67.6%) of the thioalkylated benzene **Q88**-OH as an oil.

### Scheme 87-88 step ii:

The conversion of the resulting alcohols to the corresponding iodo derivatives was performed according to the procedure described in scheme 79 -84 step iii.

**Q87**-OH was prepared analogously to **Q88**-OH.

### Synthesis of Q89-90:

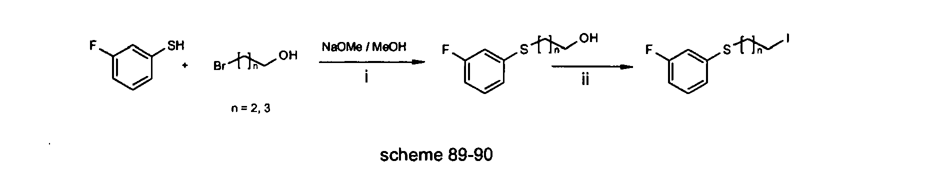

The starting phenol and alcohols were commercially available.

### Scheme 89-90 step i:

To a stirred solution of NaOMe (8.1 g, 150 mmol) in 100 ml methanol under a nitrogen atmosphere, was added dropwise 3-fluorothiophenol (9.5 ml, 100 mmol). The reaction mixture was stirred for 10 minutes at room temperature . 3-bromo-1-propanol (13.6 ml, 150 mmol) was added in one time, after which the reaction mixture was refluxed for 14 hours. After the reaction was allowed to cool, the mixture was concentrated in vacuo after which H₂O and CH₂Cl₂ were added to the residue and after separation the organic layer was dried (Na₂SO₄). The drying agent was removed by filtration and the solvent by evaporation under reduced pressure. The residue was chromatographed (SiO₂) using CH₂Cl₂/CH₃OH 99/1 as eluent to give 18.5 g (99.5%) of the alkylated product as an oil containing **Q89-**OH.

### Scheme 89-90 step ii:

The conversion of the resulting alcohols to the corresponding iodo derivatives was executed according to the procedure de scribed in scheme 79-84 step iii.

**Q90**-OH was prepared analogously to **Q89**-OH.

### Synthesis of Q96:

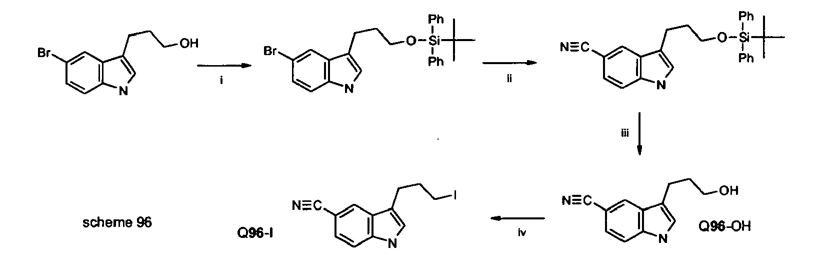

The starting 5-bromoindole alcohol was prepared according to: Campos, Kevin R.; Woo, Jacqueline C. S.; Lee, Sandra; Tillyer, Richard D., Org.Lett., 6 (2004) 79 - 82.

### Scheme 96 step i:

Was prepared according to scheme 79-84 b step i.

### Scheme 96 step ii:

A mixture under a nitrogen atmosphere of the silylated alcohol (42.4 g, 86.2 mmol), Cul (1.64 g, 8.6 mmol), Palladium tetrakis (5 g, 4.31 mmol) and potassium cyanide (11.17 g, 172.4 mmol) in 110 ml butyronitrile was refluxed for 6 h. The reaction mixture was cooled to room temperature and filtered through a pad of Hyflo. After rinsing the pad of Hyflo with 750 ml EtOAc, the organic layer was washed with H₂O (2x) and brine (1x). The organic layer was evaporated under reduced pressure and the residue was chromatographed (SiO₂) with CH₂Cl₂/petroleum ether 3/1 as eluent to give 35.6 g (94%) of the cyanated indole as a light yellow solid.

### Scheme 96 step iii:

A mixture of the cyanated indole (35.6 g, 81.1 mmol) and 105.3 ml 1.0 M TBAF (in THF) was stirred at room temperature for 20 h. The solvent was evaporated under reduced pressure and 750 ml CH₂Cl₂ was added to the residue. The CH₂Cl₂ fraction was washed with H₂O (3x). The product started to crystallize from the organic layer. The CH₂Cl₂ fractionwas separated and stirred in an ice/EtOH bath for 30 minutes . The resulting suspension was filtered by suction to give 13.7 g (72%) of the alcohol as nearly white solid.

### Scheme 96 step iv:

Was prepared according to the procedure as described for scheme 79 -84 step iii.

### Synthesis of Q97:

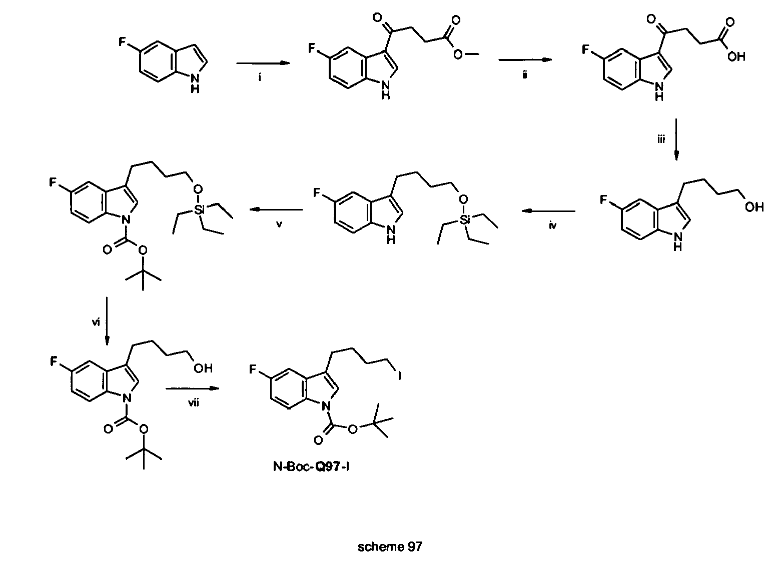

The 5-fluoroindole was commercially available.

### Scheme 97 step i:

To a cooled solution of 5-fluoroindole (3 g, 22.2 mmol) in 100 ml CH₂Cl₂ at 0 °C was added 33.8 ml 1.0 M Et₂AlCl in hexane. The resulting light yellow solution was stirred at the same temperature for 30 minutes after which a solution was added of 3-carbomethoxypropionylchloride (4.1 ml, 33.3 mmol) in 50 ml CH₂Cl₂ at 0 °C. After the complete addition the color of the solution was changed to orange and stirring was continued for another 2.5 hours at the same temperature. The reaction mixture was poured into 500 ml of Hamilton pH 7 buffer (gas evolution) and the aqueous layer was extracted with 750 ml (in total) CH₂Cl₂ (3x). The combined organic layers were washed with H₂O (1x) and brine (1x).
The CH₂Cl₂ fraction was dried (Na₂SO₄). The drying agent was removed by filtration and the solvent by evaporation under reduced pressure. The residue was chromatographed (SiO₂) with EtOAc/PA 1/1 as eluent to give 3.82 g (69%) of the acylated indole as a light colored solid.

### Scheme 97 step ii:

A mixture of the acylated indole (3.46 g, 13.9 mmol) and 28 ml 1.0 N NaOH in 75 ml THF/MeOH 2/1 was stirred at room temperature for 2.5 h. The mixture was acidified under cooling with 25 ml 1.0 N HCl. The resulting suspension was filtered by suction to give 2.42 g (74%) of the acid as an off-white solid.

### Scheme 97 step iii:

Was prepared according to the procedure as described for scheme 14 step ii.

### Scheme 97 step iv:

Was prepared according to the procedure as described for scheme (18, 51 -52, 94-95) step ii. Additionally the residue was chromatographed (SiO₂) with CH₂Cl₂ as the eluent.

### Scheme 97 step v:

To a stirring solution of the silylated alcohol (3.43 g, 10.7 mmol) in 30 ml CH₃CN was added Boc₂O (3.50 g, 16 mmol) and DMAP (0.13 g, 1.07 mmol). The yellow solution was stirred at room temperature for 30 minutes after which imidazole (0.98 g, 16 mmol) was added. Stirring was continued at room temperature for 1.5 hours after which 55 ml CH₂Cl₂ was added. The CH₂Cl₂ fraction was washed with 0.5% HCl (3x) and dried (MgSO₄). The drying agent was removed by filtration and the solvent by evaporation under reduced pressure to give 4.09 g (91%) of the carbamated indole as a thick yellow liquid.

### Scheme 97 step vi:

A mixture of the carbamated indole (4.09 g, 9.7 mmol) and 1.0 M (in THF) TBAF (12.6 ml, 12.6 mmol) was stirred at room temperature for 4 hours. H₂O and Et₂O were added. The Et₂O layer was separated and the aqueous layer was extracted with Et₂O (2x). The combined Et₂O layers were washed with H₂O (2x) and brine (1x). The Et₂O fraction was dried (by a Water Reppelling Filter) and concentrated in vacuo under reduced pressure. The residue was chromatographed (SiO₂) with CH₂Cl₂/MeOH 99:1 as eluent to give 4.04 g (87%) as a yellow oil.

### Scheme 97 step vii:

Was prepared according to the procedure as described for scheme 79-84 step iii. The iodo derivative can be coupled to an amine (see schemeA2, step i). The coupling product's Boc group can be removed by standard procedures like 1.0 M AcCl/MeOH.

Compounds **31**.HCl, **72**.HCl and **99**.HCl were made similarly to the procedure described above.

### Synthesis of Q98:

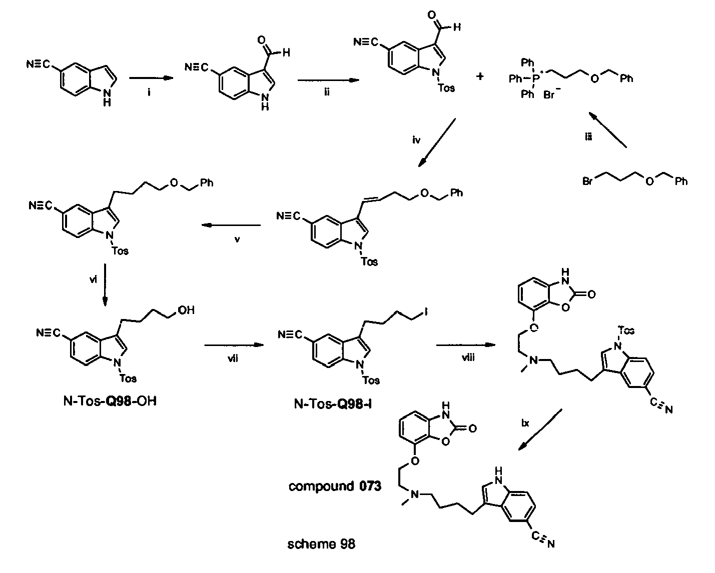

The starting material was commercially available.

### Scheme 98 step i:

POCl₃ was added dropwise to 200 ml dry DMF at 15 °C. The resulting dark pink solution was stirred for 20 min after which it was co oled to 0-5 °C. To this solution was added dropwise a solution of 5-cyanoindole (20 g, 140 mmol) in 45 ml dry DMF. After 10 min of stirring a very thick suspension was formed. The reaction mixture was allowed to warm to room temperature and stirred for 4 h. Next the reaction mixture was poured into a 650 ml saturated Na₂CO₃/ice mixture. The resulting suspension was stirred for 30 minutes after which it was filtered by suction and dried (by use of an oven) to give 29.8 g yellow solid. To this solid was adde d 80 ml EtOAc and the suspension was filtered again by suction to give 21.79 g (79%) of the formylated indole as a solid.

### Scheme 98 step ii:

A mixture of the formylated indole (1.7 g, 10.3 mmol), tosylchloride (2.99 g, 15.7 mmol) and Et₃N (25 ml, 17.99 mmol) was refluxed for 1.5 h. The resulting very thick suspension was cooled to room temperature and 35 ml ice water was added. The reaction mixture was left to stand at 4 °C for 1 hour after which the suspension was filtered by suction. The solid was furt her purified by recrystallisation from EtOAc to give 1.58 g of tosylated product.

The filtrate was evaporated under reduced pressure and the residue was chromatographed with CH₂Cl₂/PA 4:1 → CH₂Cl₂ as eluent to give 0.5 g of the tosylated product. This sample was identical and added to the previously isolatedsolid to give in total 2.08 g (64%) of the tosylated product as a white solid.

### Scheme 98 step iii:

A mixture of the bromide (40 g, 174 mmol) and PPh₃ (45.7 g, 174 mmol) was refluxed in 200 ml toluene for 16 h. After cooling to room temperature the resulting slurry (which could not be filtered), was warmed to reflux again and cooled to room temperature while the mixture was vigorously stirred. A very hard white solid crystallized from the solution. It was pulverized and filtered by suction. The solid was recrystallized from CH₃CN/petroleum ether to give 58.1 g (68%) of the corresponding phosphonium salt.

### Scheme 98 step iv:

To a stirring suspension of the phosphonium salt (41.77 g, 85 mmol) in 500 ml dry THF at -10°C was added 85 ml 1.0M (in THF) NaHMDS in 45 min.. After complete addition the reaction mixture was stirred for 1.5 hours at the same temperature. The reaction mixture was further cooled to -65°C and the tosylated formylindole (27.5 g, 84.7 mmol) was added in portions in 75 minutes by means of an addition funnel for solids. After the complete addition the reaction mixture was allowed to warm to room temperature and stirred for 22 h. 1L of ice water and 500 ml of Et₂O were added to the reaction mixture and after separation the aqueous layer was extracted with Et₂O (2x). The combined organic layers were washed with 500 ml H₂O (1x) and 400 ml brine (1x). The Et₂O fraction was evaporated under reduced pressure and the residue was chromatographed (SiO₂) with CH₂Cl₂ as the eluent to give 16.89 g (44%) of the alkene as an off-white solid.

### Scheme 98 step v:

A mixture of the alkene (11 g, 23 mmol) and 0.5 g 10% Pd/C in 240 ml EtOAc/MeOH 1/1 was hydrogenated (1 atm) at room temperature for 4 h. The reaction mixture was filtered through a pad of Hyflo which was rinsed with 200 ml EtOAc/MeOH 3/1. The filtrate was evaporated under reduced pressure to give 11.2 g (103%) as a solid.

### Scheme 98 step vi:

To a cooled clear orange solution of the tosylated indole (11.2 g, 23 mmol) in 150 ml CH₂Cl₂ at -75°C was added dropwise 100 ml 1.0M BCl₃ (in CH₂Cl₂) in 1 hour during which the temperature was kept below -60 °C. Stirring was continued at -75 °C for 2 h. The resulting pink suspension was allowed to warm to room temperature and stirred for 20 h. The reaction mixture was cooled in an ice bath and 550 ml 5% NaHCO₃ was carefully added during which the temperature was kept below 20°C and the pH rose until 8. The aqueous layer was extracted with 300 ml CH₂Cl₂ (2x). The combined organic layers were washed with H₂O (1x) and brine (1x) and dried (Na₂SO₄). The drying agent was removed by filtration and the solvent by evaporation under reduced pressure. The residue was chromatographed (SiO₂) with CH₂Cl₂/MeOH 98/2 as eluent to give 7.39 g (87%) of the de-benzylated alcohol as a solid.

### Scheme 98 step vii:

Was prepared analogously to step iv in scheme 28 -29.

### Scheme 98 step viii:

Was prepared analogously to step i in scheme A2.

### Scheme 98 step ix:

A mixture of the tosyl-indole product (0.94 g, 1.68 mmol) and 1.0M TBAF (in THF) was refluxed in 40 ml THF for 72 h. The reaction mixture was cooled to room temperature and the solvent was evaporated under reduced pressure. EtOAc was added to the residue and the organic layer was washed with H₂O (2x) and brine (1x). The EtOAc fraction was dried (MgSO₄). The drying agent was removed by filtration and the solvent by evaporation under reduced pressure. The residue was chromatographed (SiO₂) with CH₂Cl₂/MeOH/NH₃ 96/3.75/0.25 → CH₂Cl₂/MeOH/NH₃ 92/7.5/0.5 as the eluent to give 0.34 g (50%) of the de -tosylated product **73,** melting point 239-242 °C.

Compound **100** was made analogously.

### Synthesis of Q99:

**Q99**-Br was synthesized analogously to the synthesis depicted in Scheme 60 -61, using bromoethanol in the Mitsunobu step iii.

### Synthesis of Q100-101:

**Q100-**I and **Q101-**I were synthesized analogously to the synthesis depicted in scheme 19-26, steps ii, iii and iv.

### Synthesis of Q102:

**Q102-**I was synthesized as depicted in scheme 102:

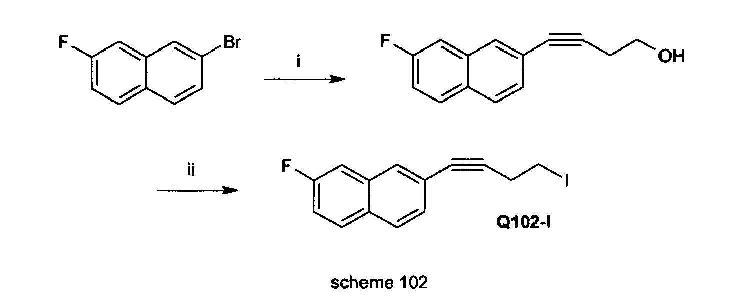

### Scheme 102, step i:

Through a suspension containing the fluorobromonapthalene (0.90 g, 4 mmol), tri - phenylphospine (0.21 g, 0.8 mmol), dichlorobis(tri -phenylphospine)palladium (0.28 g, 0.4 mmol) in 15 ml Et₃N, nitrogen was bubbled for 1 hour. 3-Butyn-1-ol (0.42 g, 0.45 ml, 6 mmol) was added and the mixture was heated to 40-50 °C on an oilbath. After 15 minutes of stirring at this temperature, Cul (0.15 g, 0.8 mmol) was added and the mixture was heated at 70 °C and stirred for 48 hours.
The resulting black suspension was allowed to reach room temperature and diethyl ether and water were added. The fractions were separated and the water layer was extracted twice with diethyl ether. The combined organic extracts were washed with water, brine and dried (Na₂SO₄). After removal of the drying agent by filtration and solvent by concentration *in vacuo,* the residu was subjected to flash chromatography (SiO₂, eluent: DCM) affording **Q102-**OH, 4-(2-fluoro-napthalene-7-yl)-3-butyne-1-ol (0.30g, 1.46 mmol).

### Scheme 102, step ii:

The conversion of the alcohol of step i to the corresponding iodo -derivative was performed according to scheme 79-84 step iii, yielding **Q102-**I**.**

### Synthesis of Q103:

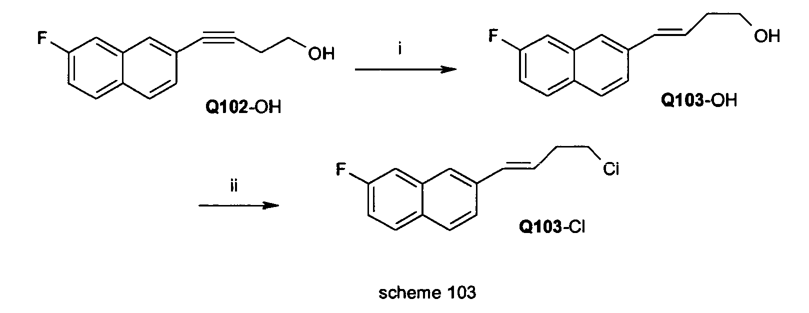

### Scheme 103, step i:

A solution of Red-Al (4.47 ml of a 3.4 M solution in toluene) in 25 ml of dry diethyl ether was cooled in an ice-bath under nitrogen to which a solution of **Q102**-OH (1.90 g, 9.5 mmol) in 40 ml of diethylther (dry) was added dropwise. After the addition was complete, the resulting mixture is stirred for 10 min at 0 °C after which it was allowed to reach room temperature and stirred for an additional 2.5 hours. The reaction mixture was again cooled in a ice -bath and quenched by the careful addition of 50 ml of 3.6 M H₂SO₄. The reaction mixture was extracted three times with diethyl ether. The combined organic extracts are washed with water, brine, and dried Na₂SO₄). After removal of the drying agent by filtration and solvent by concentration *in vacuo,* the residu was subjected to flash chromatography (SiO₂, eluent: DCM) affording 1.17 g of **Q103**-OH, 4-(2-fluoro-naphtalene-7-yl)-3-butene-1-ol (5.8 mmol).

### Scheme 103, step ii:

5 ml of concentrated hydrochloric acid is added to a solution of **Q103**-OH (1.17 g, 5.8 mmol) in 5 ml of THF. The mixture is stirred for 4.5 hours at room temperature after which another 2 ml of concentrated hydrochloric acid and 2 ml of THF are added. After another 30 minutes diethyl ether and water are added and the resulting fractions were separated. The water layer is extracted twice with diethyl ether. The combined organic fractions are washed with water, brine, dried (Na₂SO₄). After removal of the drying agent by filtration and solvent by concentration *in vacuo,* the residu was subjected to flash chromatography (SiO_{2,} eluent: DCM) affording 1.03 g of **Q103**-Cl (4.67 mmol).

### Synthesis of Q104:

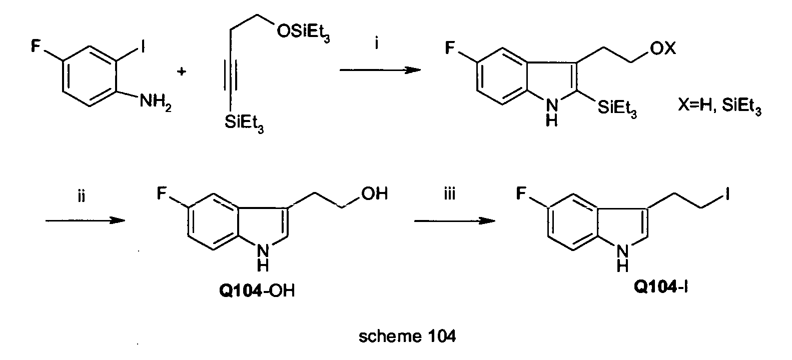

### Scheme 104, step i:

2-iodo-4-fluoroaniline (2.70 g,11.4 mmol), 4-triethylsilyl-1-(triethylsilyloxy)-3-butyne (3.82 g, 12.5 mmol), LiCl (0.48 g, 11.4 mmol), Na₂CO₃ (2.18 g, 20.5 mmol), Pd(OAc)₂ (0.128 g, 0.57 mmol) were suspended in 120 ml DMF and nitrogen was bubbled through the suspension for 45 minutes . The mixture was heated to 100 °C in an oilbath and stirred at this temperature for 16 hours after which it was allowed to reach room temperature and subsequently concentrated *in vacuo.* The residu was taken up in some dichloromethane and filtered over Celite. Flash chromatography on silica (eluent: diethyl ether/petroleum ether 1/3) afforded a mixture of unprotected and triethylsilyl protected 2-triethylsilyl-5-fluoro-tryptophol (2.09 g, 6.02 mmol).

### Scheme 104, step ii:

A mixture of unprotected and triethylsilyl protected 2-triethylsilyl-5-fluoro-tryphtol (2.74 g, 7.83 mmol) and 15.7 ml of a 1.0 N solution of TB AF in THF were stirred for 48 hours at room temperature. Diethyl ether and water were added and the fractions were separated. The water layer was extracted twice with diethyl ether. The combined organic extracts were washed with water, brine and dried (Na₂SO₄). After removal of the drying agent by filtration and solvent by concentration *in vacuo,* the residu was subjected to flash chromatography (SiO₂, eluent: DCM/MeOH 97/3) affording **Q104**-OH, 5-fluoro-tryptophol (1.14g, 6.36 mmol).

### Scheme 104, step iii:

The conversion of the alcohol **Q104**-OH to the corresponding iodo-derivate was performed according to the synthesis given in scheme 79-84 step iii, yielding **Q104-**I.

The specific compounds of which the synthesis is described above are intended to further illustrate the invention in more detail, and therefore are not deemed to restrict the scope of the invention in any way. Other embodiments of the invention will be apparent to those skilled in the art from consideration of the specification and practice of the invention disclosed herein. It is thus intended that the specification and examples be considered as exemplary only, with a true scope and spirit of the invention being indicated by the claims.

### ABBREVIATIONS

- AcCl: acetylchloride
- ADDP: 1,1'-(azodicarbonyl)dipiperidine
- CDI: carbonyldiimidazol
- Dba: see Huang et al., J. Am.Chem.Soc., 125(2003)6653
- DCE: dichloroethane
- DCM: dichloromethane
- DIAD: diisopropyldiazodicarboxylate
- DIPE: diisopropylether
- DIPEA: diisopropylethylamine

| | CH₂Cl₂(ml) | MeOH(ml) | NH₄OH(ml) |
|---|---|---|---|
| DMA 0.125 | 980 | 18.75 | 1.25 |
| DMA 0.187 | 970 | 28.13 | 1.87 |
| DMA 0.25 | 960 | 37.5 | 2.5 |
| DMA 0.50 | 920 | 75.0 | 5.0 |
| DMA 0.75 | 880 | 112.5 | 7.5 |
| DMA 1.00 | 840 | 150.0 | 10.0 |

- DMAP: 4-dimethylaminopyridin
- DME: dimethoxyethane
- DMF: N,N-dimethylformamide
- EtOH: ethanol
- MeOH: methanol
- MTBE: methyl(*tert*.)-butylether
- NMP: N-methylpyrrolidon
- PA: petroleum ether
- TBAB: tetrabutylammoniumbromide
- TBAC: tetrabutylammoniumchloride
- TBAF: tetrabutylammoniumfluoride
- THF: tetrahydrofurane
- XPHOS: see Huang et al., J. Am.Chem.Soc., 125(2003)6653

### EXAMPLE: FORMULATION OF COMPOUND 16 USED IN ANIMAL STUDIES

**For oral *(p.o.)* administration** : to the desired quantity (0.5-5 mg) of the solid compound 16 in a glass tube, some glass beads were added and the solid was milled by vortexing for 2 minutes. After addition of 1 ml of a solution of 1% methylcellulose in water and 2% (v/v) of Poloxamer 188 (Lutrol F68), the compound was suspended by vortexing for 10 minutes. The pH was adjusted to 7 with a few drops of aqueous NaOH (0.1N). Remaining particles in the suspension were further suspended by using an ultrasonic bath.

**For intraperitoneal *(i.p.)* administration:** to the desired quantity (0.5-15 mg) of the solid compound 16 in a glass tube, some glass beads were added and the solid was milled by vortexing for 2 minutes. After addition of 1 ml of a solution of 1% methylcellulose and 5% mannitol in water, the compound was suspended by vortexing for 10 minutes. Finally the pH was adjusted to 7.

### EXAMPLE: PHARMACOLOGICAL TESTRESULTS

**Table 2. In vitro affinities and functional activity of compounds of the invention**

| | | | |
|---|---|---|---|
| Dopamine-D₂ and serotonin reuptake receptor affinity data obtained according to the protocols given above are shown in the table below. *In vitro* functional activity at cloned human dopamine D_{2,L} receptors as measured by accumulation of radiolabeled cAMP (potency: pEC_{50,} intrinsic activity **ε**) | | | |

| | **Dopamine-D₂** | **5-HT reuptake** | **Dopamine-D₂** |
|---|---|---|---|
| | | | |

| | **binding** | **binding** | **cAMP accum** |
|---|---|---|---|
| | | | |

| **No** | **pKₗ** | **pKₗ** | **ε** (intrinsic activity) |
|---|---|---|---|
| | | | |
| **11** | **7.9** | **7.4** | **0.57** |
| **13** | **8.2** | **8.2** | **0.81** |
| **14** | **8.7** | **8.4** | **0.88** |
| **16** | **8.4** | **7.4** | **0.39** |
| **33** | **8.0** | **8.4** | **0.20** |
| **44** | **7.8** | **8.1** | **0.16** |
| **94** | **7.0** | **8.4** | **0.41** |
| **106** | **7.6** | **>9.0** | **0.23** |

## Claims

1. Compounds of the general formula (1):
**Z-T-Ar** (1)
wherein:
Z is a fragment of the general formula (2) 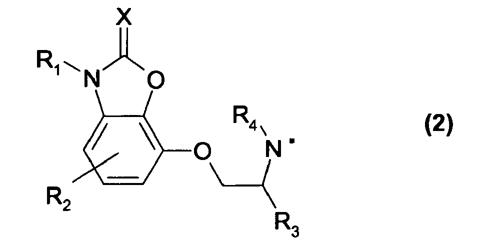 wherein: X=S or O,
R₁ is H, (C₁-C₆)alkyl, CF₃, CH₂CF₃, OH or O-(C₁-C₆)alkyl
R₂ is H, (C₁-C₆)alkyl, halogen or cyano
R₃ is H or (C₁-C₆)alkyl
R₄ is H, (C₁-C₆)alkyl optionally substituted with a halogen atom
T is a saturated or unsaturated carbon chain of 2-7 atoms, wherein one carbon atom may be replaced with a nitrogen atom, optionally substituted with an (C₁-C₃)- alkyl, CF₃ or CH₂CF₃ group, or an oxygen atom or a sulphur atom, and which chain is optionally substituted with one or more substituents selected from the group consisting of (C₁-C₃)alkyl, (C₁-C₃)alkoxy, halogen, cyano, trifluoromethyl, OCF₃, SCF₃, OCHF₂ and nitro,
Ar is selected from the groups: 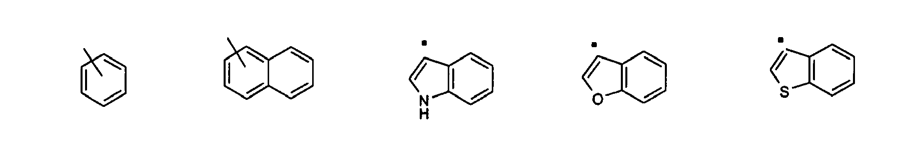 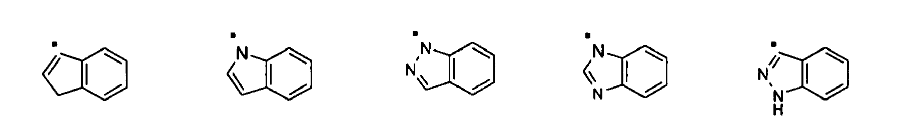
which Ar group is optionally further substituted with one or more substituents selected from the group consisting of (C₁-C₃)alkyl, (C₁-C₃)alkoxy, halogen, cyano, trifluoromethyl, OCF₃, SCF₃ OCHF₂ and nitro,
and in which Ar groups that contain a five-membered ring, the double bond in the five-membered ring may be saturated,
and tautomers, stereoisomers and N-oxides thereof, as well as pharmacologically acceptable salts, hydrates and solvates of said compounds of formula (1) and its tautomers, stereoisomers and N -oxides.

2. Compounds of the formula (1) as claimed in claim 1, wherein Z is selected from the group consisting of: 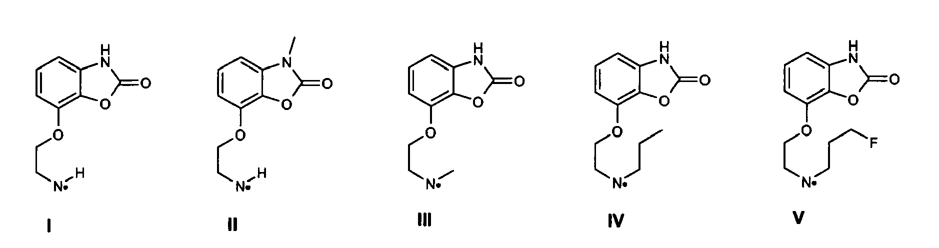 and wherein the second part of the molecule, represented by the symbols -T-Ar in formula (1), is selected from the group consisting of: 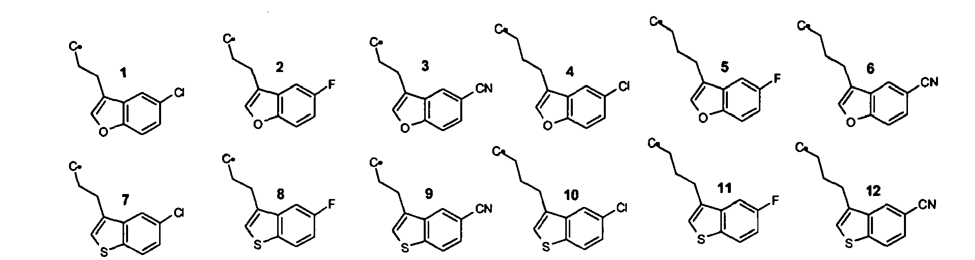 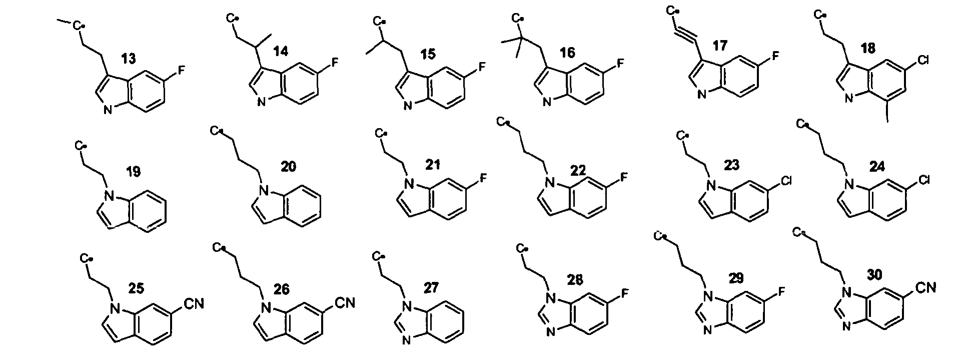 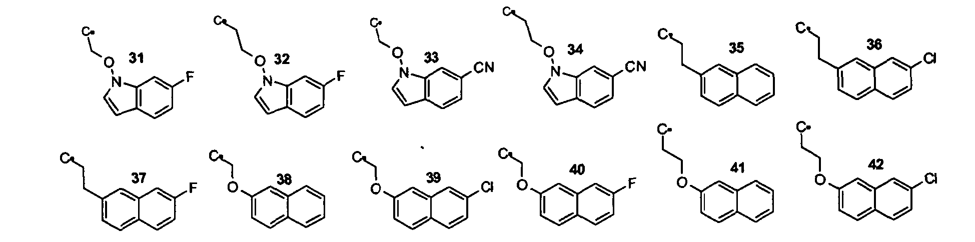 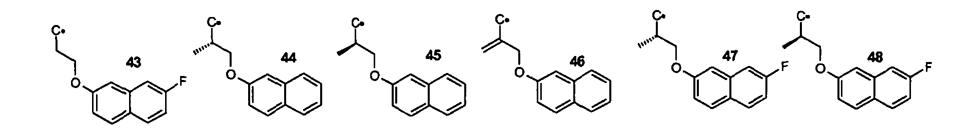 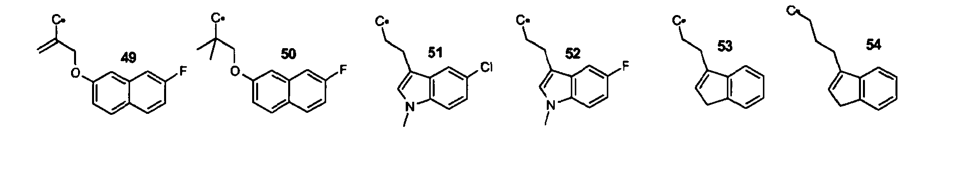 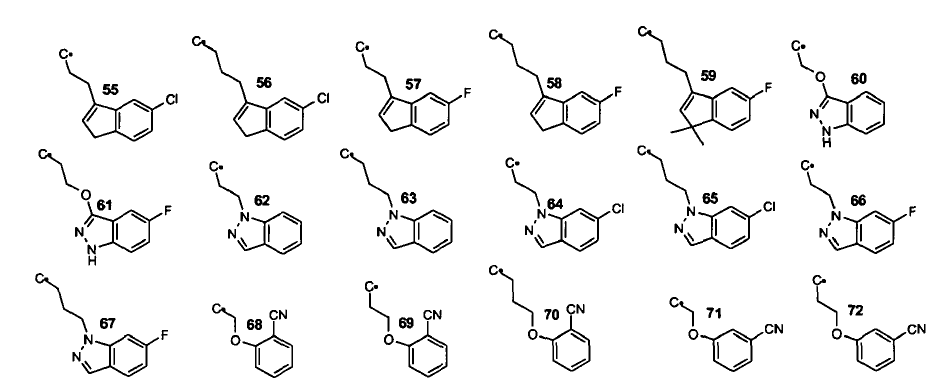 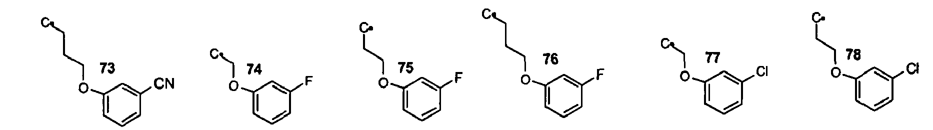 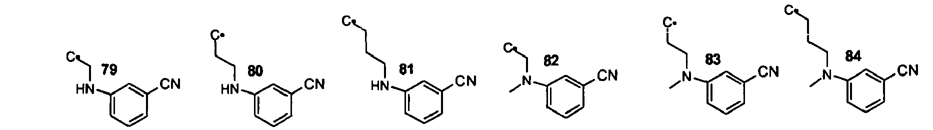 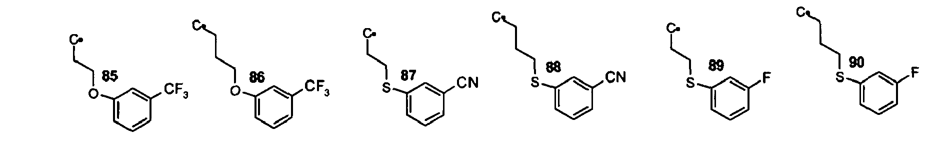 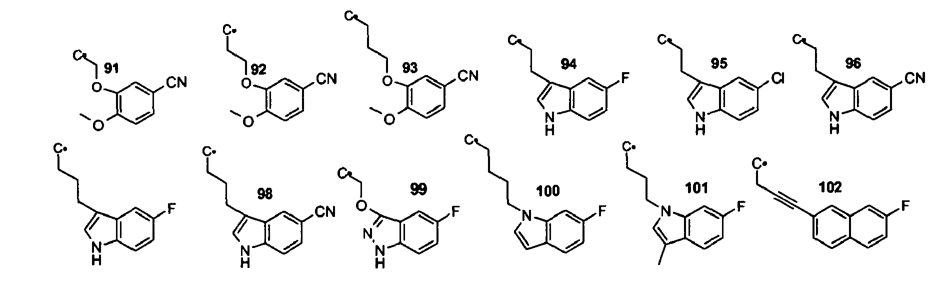 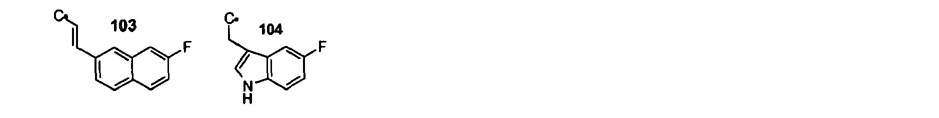 and tautomers, stereoisomers and N-oxides thereof, as well as pharmacologically acceptable salts, hydrates and solvates of said compounds of formula (1) and its tautomers, stereoisomers and N -oxides.

3. A pharmaceutical composition comprising, in addition to a pharmaceutically acceptable carrier and/or at least one pharmaceutically acceptable auxiliary substance, a pharmacologically active amount of at least one compound of one of the claims 1-2, or a salt thereof, as an active ingredient.

4. A method of preparing a composition as claimed in claim 3, **characterised in that** at least one compound of one of the claims 1 -2, or a salt thereof, is brought into a form suitable for administration

5. A compound as claimed in one of the claims 1-2, or a salt thereof, for use as a medicament.

6. Use of a compound as claimed in of one of the claims 1-2, for the preparation of a pharmaceutical composition for the treatment of CNS disorders.

7. Use as claimed in claim 6, **characterized in that** said disorders are aggression, anxiety disorders, autism, vertigo, depression, disturbances of cognition or memory, Parkinson's disease, schizophrenia and other psychotic disorders.

8. Use as claimed in claim 6, **characterized in that** said disorder is depression.

9. Use as claimed in claim 6, **characterized in that** said disorders are schizophrenia and other psychotic disorders.

10. Use as claimed in claim 6, **characterized in that** said disorder is Parkinson's disease.

## Patentansprüche

1. Verbindungen der allgemeinen Formel (1):
**Z-T-Ar** (1),
worin:
Z ein Fragment der allgemeinen Formel (2) 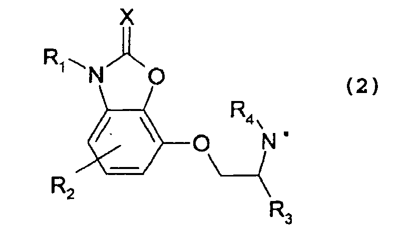 darstellt, worin:
X = S oder O,
R₁ für H, (C₁-C₆)Alkyl, CF₃, CH₂CF₃, OH oder O-(C₁-C₆)Alkyl steht,
R₂ für H, (C₁-C₆)Alkyl, Halogen oder Cyano steht,
R₃ für H oder (C₁-C₆)Alkyl steht,
R₄ für H, (C₁-C₆)Alkyl steht, gegebenenfalls substituiert mit einem Halogenatom,
T eine gesättigte oder ungesättigte Kohlenstoffkette mit 2-7 Atomen darstellt, worin ein Kohlenstoffatom ersetzt sein kann mit einem Stickstoffatom, gegebenenfalls substituiert mit einer (C₁-C₃)-Alkyl-, CF₃- oder CH₂CF₃-Gruppe, oder mit einem Sauerstoffatom oder einem Schwefelatom, und welche Kette gegebenenfalls substituiert ist mit einem oder mehreren Substituenten, ausgewählt aus der Gruppe bestehend aus (C₁-C₃)Alkyl, (C₁-C₃)Alkoxy, Halogen, Cyano, Trifluormethyl, OCF₃, SCF₃, OCHF₂ und Nitro,
Ar ausgewählt wird aus den Gruppen: 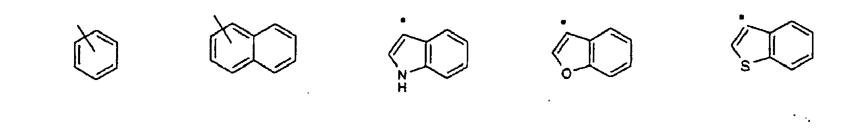 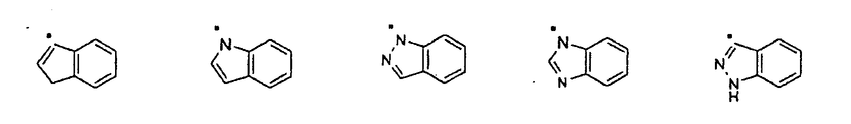
welche Ar-Gruppe gegebenenfalls weiter substituiert ist mit einem oder mehreren Substituenten, ausgewählt aus der Gruppe bestehend aus (C₁-C₃)Alkyl, (C₁-C₃)Alkoxy, Halogen, Cyano, Trifluormethyl, OCF₃, SCF₃ OCHF₂ und Nitro,
und in welchen Ar-Gruppen, die einen fünfgliedrigen Ring enthalten, die Doppelbindung in dem fünfgliedrigen Ring gesättigt sein kann,
und Tautomere, Stereoisomere und N-Oxide davon, als auch pharmakologisch annehmbare Salze, Hydrate und Solvate von besagten Verbindungen der Formel (1) und ihre Tautomere, Stereoisomere und N-Oxide.

2. Verbindungen der Formel (1) wie in Anspruch 1 beansprucht, worin Z ausgewählt wird aus der Gruppe bestehend aus: 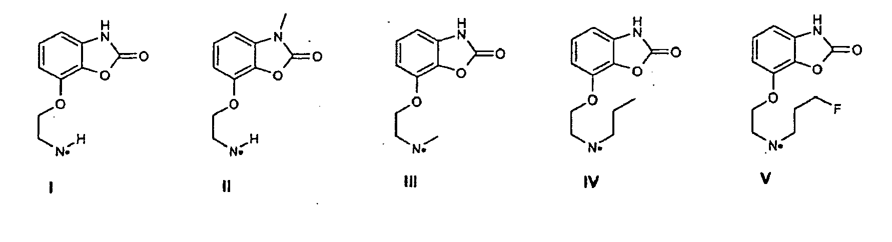 und worin der zweite Teil des Moleküls, dargestellt durch die Symbole -T-Ar in Formel (1), ausgewählt wird aus der Gruppe bestehend aus: 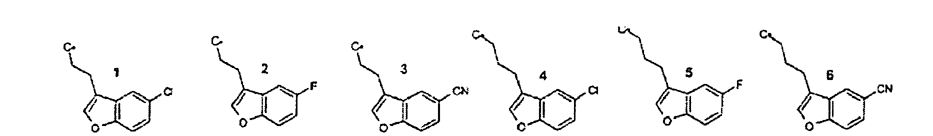 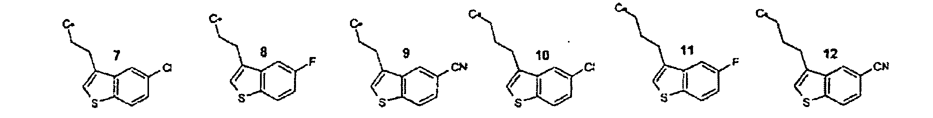 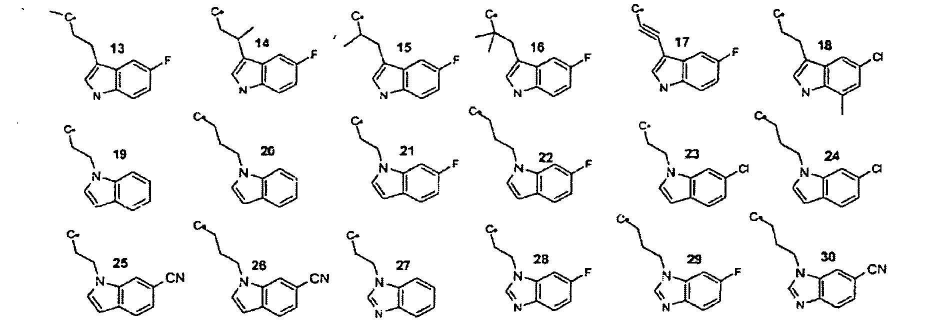 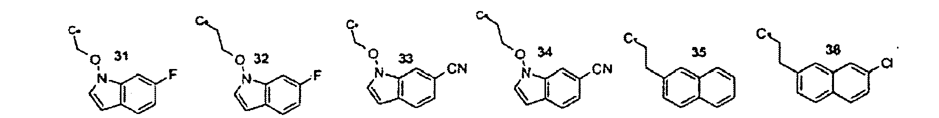 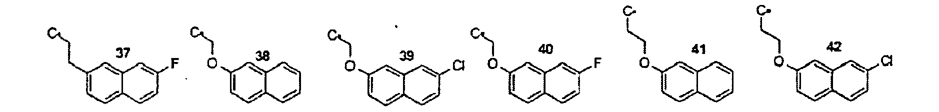 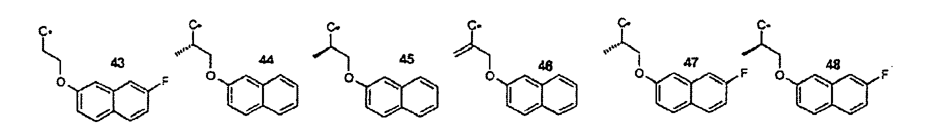 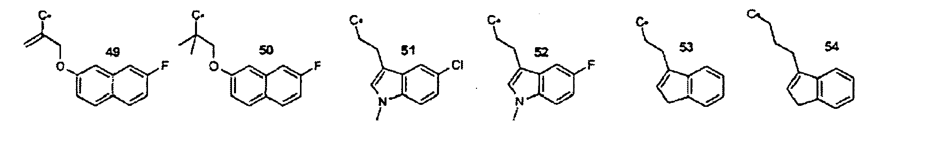 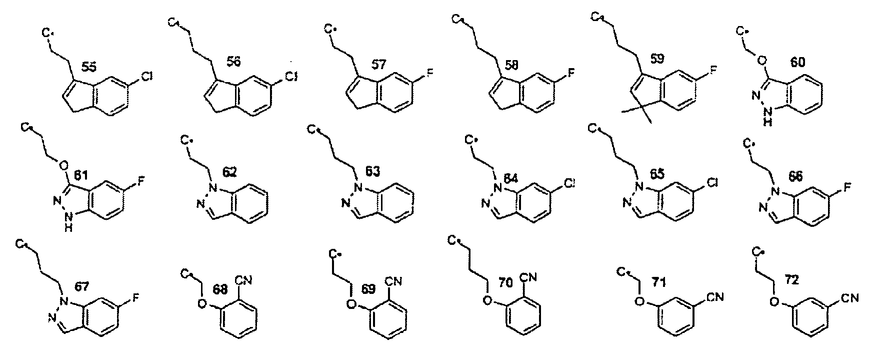 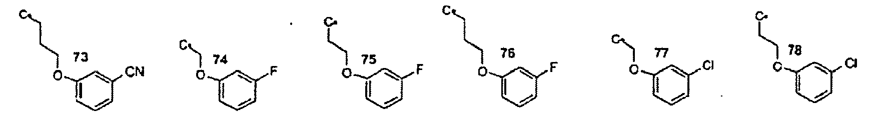 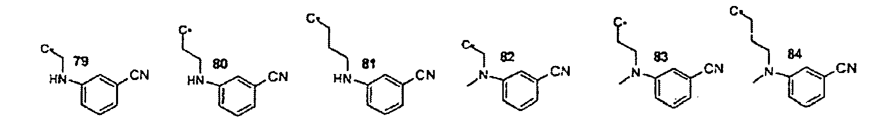 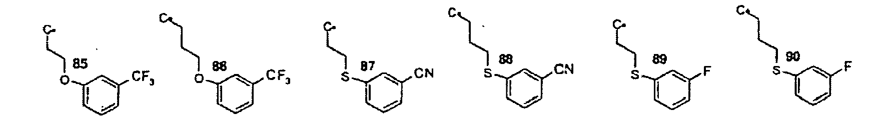 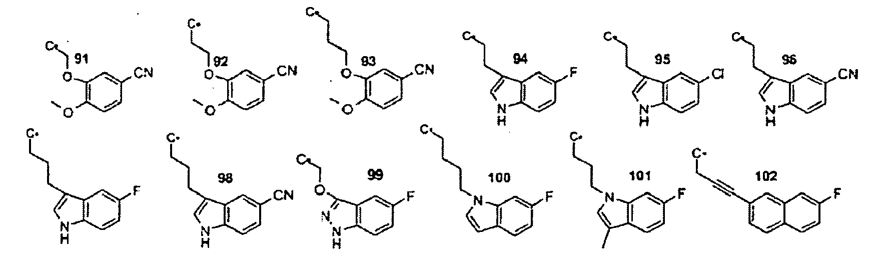 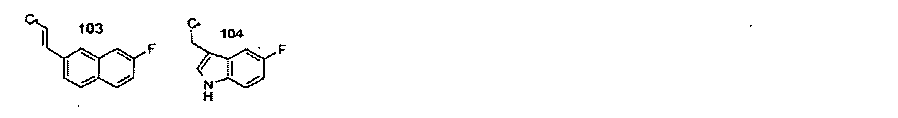 und Tautomere, Stereoisomere und N-Oxide davon, als auch pharmakologisch annehmbare Salze, Hydrate und Solvate von besagten Verbindungen der Formel (1) und ihre Tautomere, Stereoisomere und N-Oxide.

3. Pharmazeutische Zusammensetzung, umfassend zusätzlich zu einem pharmazeutisch annehmbaren Träger und/oder mindestens einer pharmazeutisch annehmbaren Hilfssubstanz eine pharmakologisch wirksame Menge von mindestens einer Verbindung nach einem der Ansprüche 1-2, oder ein Salz davon, als wirksamen Inhaltsstoff.

4. Verfahren zum Herstellen einer Zusammensetzung wie in Anspruch 3 beansprucht, **dadurch gekennzeichnet, dass** mindestens eine Verbindung nach einem der Ansprüche 1-2 oder ein Salz davon in eine für Verabreichung geeignete Form gebracht wird.

5. Verbindung wie in einem der Ansprüche 1-2 beansprucht oder ein Salz davon zur Verwendung als Medikament.

6. Verwendung einer Verbindung wie in einem der Ansprüche 1-2 beansprucht für die Herstellung einer pharmazeutischen Zusammensetzung für die Behandlung von CNS-Störungen.

7. Verwendung wie in Anspruch 6 beansprucht, **dadurch gekennzeichnet, dass** besagte Störungen Aggression, Angststörungen, Autismus, Schwindel, Depression, Störungen von Kognition oder Gedächtnis, Parkinson-Krankheit, Schizophrenie und andere psychotische Störungen sind.

8. Verwendung wie in Anspruch 6 beansprucht, **dadurch gekennzeichnet, dass** besagte Störung Depression ist.

9. Verwendung wie in Anspruch 6 beansprucht, **dadurch gekennzeichnet, dass** besagte Störungen Schizophrenie und andere psychotische Störungen sind.

10. Verwendung wie in Anspruch 6 beansprucht, **dadurch gekennzeichnet, dass** besagte Störung Parkinson-Krankheit ist.

## Revendications

1. Composés de formule générale (1) :
Z-T-Ar (1)
où :
Z est un fragment de formule générale (2) 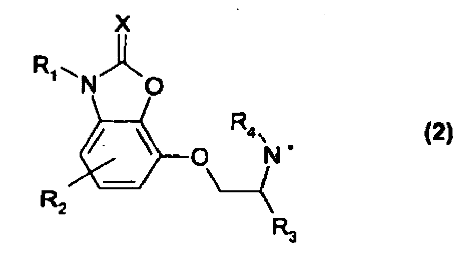 où : X = S ou O,
R₁ est H, (C₁-C₆)alkyle, CF₃, CH₂CF₃, OH ou O-(C₁-C₆)alkyle
R₂ est H, (C₁-C₆)alkyle, halogène ou cyano
R₃ est H ou (C₁-C₆)alkyle
R₄ est H, (C₁-C₆)alkyle éventuellement substitué avec un atome d'halogène
T est une chaîne carbonée saturée ou insaturée de 2-7 atomes, où un atome de carbone peut être remplacé par un atome d'azote, éventuellement substitué avec un groupe (C₁-C₃)alkyle, CF₃ ou CH₂CF₃, ou un atome d'oxygène ou un atome de soufre, et laquelle chaîne est éventuellement substituée avec un ou plusieurs substituants choisis dans le groupe consistant en (C₁-C₃)alkyle, (C₁-C₃)alcoxy, halogène, cyano, trifluorométhyle, OCF₃, SCF₃, OCHF₂ et nitro,
Ar est choisi parmi les groupes : 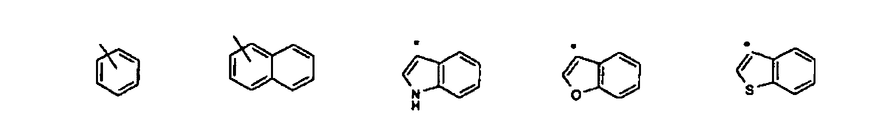 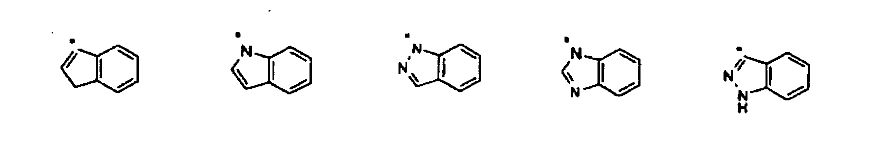
lequel groupe Ar est éventuellement substitué encore avec un ou plusieurs substituants choisis dans le groupe consistant en (C₁-C₃)alkyle, (C₁-C₃)alcoxy, halogène, cyano, trifluorométhyle, OCF₃, SCF₃, OCHF₂ et nitro,
et dans les groupes Ar qui contiennent un cycle à cinq chaînons, la double liaison dans le cycle à cinq chaînons peut être saturée,
et leurs tautomères, stéréoisomères et N-oxydes, ainsi que les sels, hydrates et solvates pharmacologiquement acceptables desdits composés de formule (1) et de leurs tautomères, stéréoisomères et N-oxydes.

2. Composés de formule (1) selon la revendication 1, où Z est choisi dans le groupe consistant en : 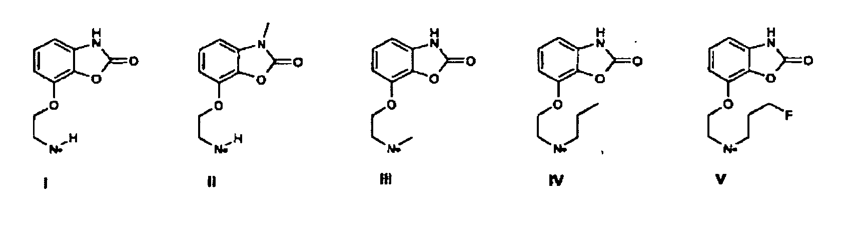 et où la seconde partie de la molécule, représentée par les symboles -T-Ar dans la formule (1), est choisie dans le groupe consistant en : 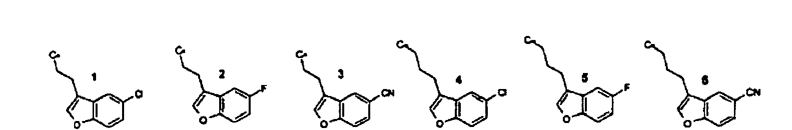 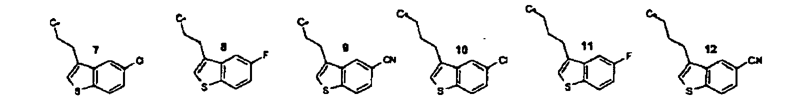 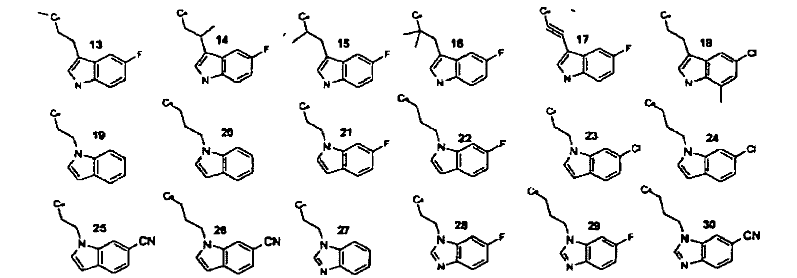 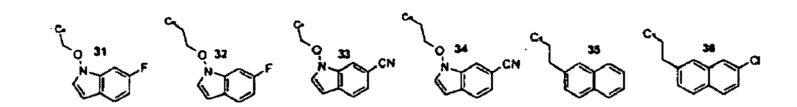 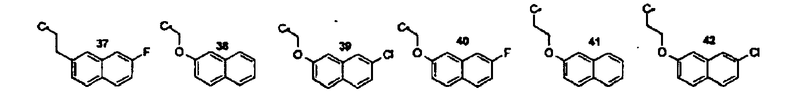 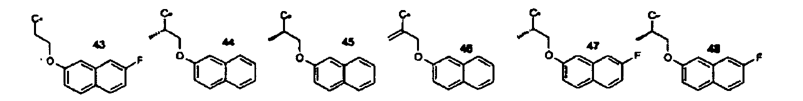 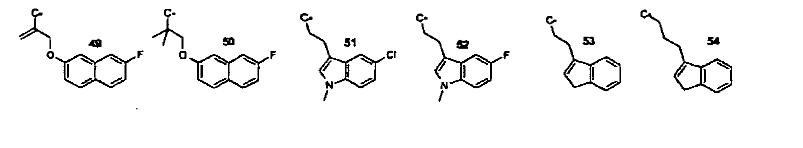 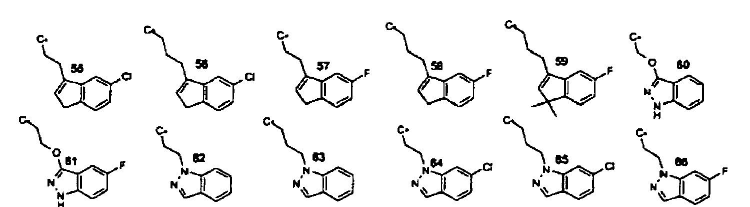 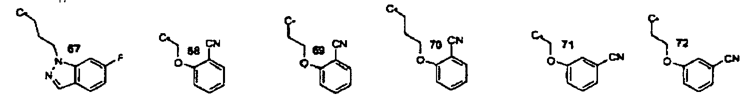 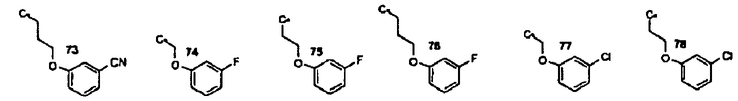 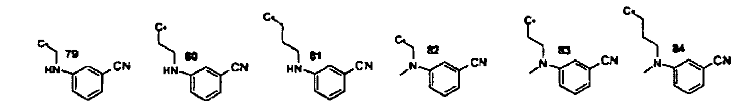 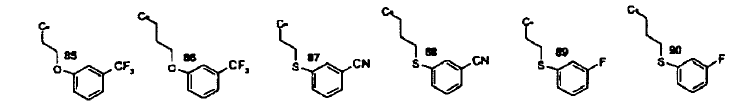 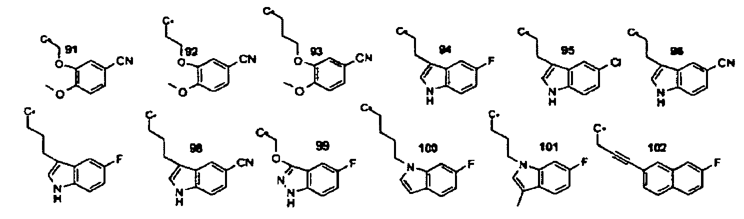 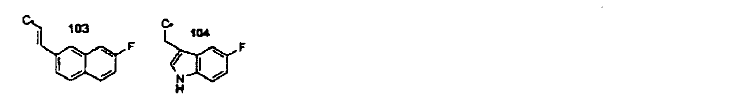 et leurs tautomères, stéréoisomères et N-oxydes ainsi que les sels, hydrates et solvates pharmacologiquement acceptables desdits composés de formule (1) et de leurs tautomères, stéréoisomères et N-oxydes.

3. Composition pharmaceutique comprenant, en plus d'un vecteur pharmaceutiquement acceptable et/ou d'au moins une substance auxiliaire pharmaceutiquement acceptable, une quantité pharmacologiquement active d'au moins un composé de l'une des revendications 1-2, ou d'un sel de celui-ci, comme ingrédient actif.

4. Procédé de préparation d'une composition selon la revendication 3, **caractérisé en ce qu'**au moins un composé de l'une des revendications 1-2, ou un sel de celui-ci, est incorporé dans une forme appropriée pour l'administration.

5. Composé selon l'une des revendications 1-2, ou sel de celui-ci, destiné à être utilisé comme médicament.

6. Utilisation d'un composé selon l'une des revendications 1-2 pour la préparation d'une composition pharmaceutique pour le traitement des troubles du SNC.

7. Utilisation selon la revendication 6, **caractérisée en ce que** lesdits troubles sont l'agression, les troubles de type anxiété, l'autisme, le vertige, la dépression, les perturbations de la cognition ou de la mémoire, la maladie de Parkinson, la schizophrénie et d'autres troubles psychotiques.

8. Utilisation selon la revendication 6 **caractérisée en ce que** ledit trouble est la dépression.

9. Utilisation selon la revendication 6 **caractérisée en ce que** lesdits troubles sont la schizophrénie et d'autres troubles psychotiques.

10. Utilisation selon la revendication 6 **caractérisée en ce que** ledit trouble est la maladie de Parkinson.
